(19) Europäisches Patentamt / European Patent Office / Office européen des brevets

(11) **EP 4 588 469 A1**

(12) **EUROPEAN PATENT APPLICATION**
published in accordance with Art. 153(4) EPC

(43) Date of publication:
**23.07.2025 Bulletin 2025/30**

(21) Application number: **23864787.9**

(22) Date of filing: **15.09.2023**

(51) International Patent Classification (IPC):
*A61K 9/00* (2006.01)     *A61P 27/02* (2006.01)
*A61K 47/26* (2006.01)     *A61K 9/08* (2006.01)
*A61K 38/17* (2006.01)

(52) Cooperative Patent Classification (CPC):
A61K 9/00; A61K 9/08; A61K 38/17; A61K 47/26;
A61P 27/02

(86) International application number:
**PCT/CN2023/119034**

(87) International publication number:
**WO 2024/056058 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 16.09.2022  CN 202211134160
              20.09.2022  CN 202211141413
              07.09.2023  CN 202311153498

(71) Applicant: **QILU PHARMACEUTICAL CO., LTD.
Jinan, Shandong 250100 (CN)**

(72) Inventors:
- **ZHANG, Le**
  **Jinan, Shandong 250100 (CN)**
- **LIU, Jun**
  **Jinan, Shandong 250100 (CN)**
- **AN, Zhenming**
  **Jinan, Shandong 250100 (CN)**

- **CONG, Riyuan**
  **Jinan, Shandong 250100 (CN)**
- **ZHOU, Bingbing**
  **Jinan, Shandong 250100 (CN)**
- **YANG, Chen**
  **Jinan, Shandong 250100 (CN)**
- **SUN, Lixia**
  **Jinan, Shandong 250100 (CN)**
- **WANG, Qingmin**
  **Jinan, Shandong 250100 (CN)**
- **ZHENG, Huanlan**
  **Jinan, Shandong 250100 (CN)**
- **WANG, Yatao**
  **Jinan, Shandong 250100 (CN)**

(74) Representative: **Schmitz, Joseph
Isler & Pedrazzini AG
Giesshübelstrasse 45
Postfach 1772
8027 Zürich (CH)**

(54) **STABLE HIGH-CONCENTRATION SELF-BUFFERING PHARMACEUTICAL COMPOSITION**

(57)     Provided is a stable high-concentration self-buffering pharmaceutical composition, comprising a VEGF binding molecule, a stabilizer, a surfactant, and the like. The pH of the composition is 5.0-7.0. The pharmaceutical composition features no additionally added buffering agent, very high stability, convenient storage, and wide application prospects.

FIG.1

**Description**

The present application claims priorities to Chinese Patent Application No. CN202211134160.6, filed before the China National Intellectual Property Administration (CNIPA) on September 16, 2022, titled "STABLE HIGH-CONCENTRATION SELF-BUFFERING PHARMACEUTICAL COMPOSITION", Chinese Patent Application No. CN202211141413.2, filed before the China National Intellectual Property Administration (CNIPA) on September 20, 2022, titled "STABLE HIGH-CONCENTRATION SELF-BUFFERING PHARMACEUTICAL COMPOSITION", and Chinese Patent Application No. CN202311153498.0, filed before the China National Intellectual Property Administration (CNIPA) on September 07, 2023, titled "STABLE HIGH-CONCENTRATION SELF-BUFFERING PHARMACEUTICAL COMPOSITION", which are incorporated herein by reference in their entirety.

**TECHNICAL FIELD**

The present application relates to the field of pharmaceutical compositions, in particular to a stable high-concentration self-buffering pharmaceutical composition.

**BACKGROUND**

Vascular endothelial growth factor (VEGF) is a known regulator of angiogenesis and neovascularization, and has been proved to be a key factor in the formation of tumors and eye diseases or disorders. Many VEGF binding molecules, including a VEGF receptor fusion protein or a VEGF antibody, have been approved for therapeutic use in humans and other mammals. For example, EYLEA® (Regeneron Pharmaceuticals, Inc.), an anti-VEGF drug, reduces vascular permeability and further inhibits neovascularization by closely binding to VEGF. Since August 2014, the Food and Drug Administration (FDA) and the European medicines agency (EMA) have successively approved EYLEA® for the treatment of indications such as macular edema secondary to retinal vein occlusion, wet age-related macular degeneration, and diabetic macular edema by intravitreal injection.

It is regrettable that the prior art still has many shortcomings and needs to be improved. For example, the concentration of a therapeutic antibody in a pharmaceutical formulation varies widely according to different administration routes. When the volume of injection is limited, it is usually necessary to develop a high-concentration antibody formulation. For example, it is often necessary to develop a high-concentration formulation for intravitreal injection or subcutaneous administration. At the same time, the preparation of a protein drug can itself pose many challenges for pharmaceutical scientists. Thus, it is necessary to find a formulation that stabilizes a protein drug and makes it resistant to degradation, aggregation, misfolding, etc. caused by proteolysis, denaturation, etc. In particular, it may be challenging to find a suitable stability condition for an engineered protein which is different from a known protein in substance. It is also desirable to have a protein drug in a form convenient for a patient. Desired characteristics include stability at ambient and refrigerated temperature, suitability for long-term storage, appropriate administration time and dosage, and minimization of discomfort after administration.

Although a formulation suitable for intravitreal administration has been found in the prior art, there is still a need for a stable liquid formulation of high concentration VEGF receptor fusion protein suitable for ocular administration. The formulation containing high concentration VEGF receptor fusion protein can reduce a volume of administration, shorten an injection time of an eye, increase administration dosage, prolong administration interval, reduce frequency of administration, and improve an efficiency of manufacture and storage. At the same time, studies have also found that many commonly used buffering agents for preparing drug proteins, especially for ocular drugs, have certain limitations and risks, which may affect safety of drugs.

A buffering agent such as acetate, succinate, citrate, histidine (imidazole), phosphate, and Tris, which has been found to have undesirable limitations and disadvantages when used in the preparation of a protein drug. Specifically, additional components in the formulation complicate the preparation process, increase harmful components, affect the overall stability and shelf life of drugs, and increase the risk of end-user acceptability. It is well known in the art that although there is a system without buffer salt in the prior art, it is challenging to obtain an effective pharmaceutical composition without buffer salt system for specific drugs, and the result is often unpredictable, the technical difficulty of high-concentration self-buffering system is even higher. At the same time, it is challenging to improve the stability of pharmaceutical composition in industry. From a perspective of formulation, high concentration of a protein leads to a higher aggregation rate of the protein, because closer contact between different components can lead to intermolecular attraction and self-aggregation events. It is also a challenge to obtain pH and an adjuvant concentration that meet the requirements of UF/DF step due to size exclusion and Daonan equilibrium effect, and higher protein concentration intensifies this challenge. Thus, it is necessary to continue optimizing the buffering system and develop a liquid formulation of a stable high concentration VEGF receptor fusion protein suitable for ocular administration.

## SUMMARY

One of the purposes of the present application is to provide an improved high-concentration pharmaceutical composition of a VEGF binding molecule with high concentration and without additional buffering agent, *i.e.*, the present application provides a high-concentration protein self-buffering pharmaceutical composition without a buffering salt system. Through years of exploration and experimentation, the inventors finally prepared and obtained a high-concentration self-buffering aqueous pharmaceutical composition of a VEGF binding molecule. In addition, the present application also surprisingly found that the pharmaceutical composition with a high-concentration self-buffering system also has high stability and broad application prospects.

The present application discloses a self-buffering high-concentration aqueous pharmaceutical composition of a VEGF binding molecule. The high-concentration system per se is easy to an aggregation event making the formulation become unstable, especially for the self-buffering high-concentration system. Generally, the self-buffering high-concentration system is difficult to store at room temperature, and the aggregation event is extremely easy to occur. An aflibercept pharmaceutical composition disclosed in the present application is stable and has a sufficient shelf life for the production, storage and dispensing of a drug. For example, the self-buffering high-concentration aflibercept pharmaceutical composition disclosed in the present application has a shelf life of at least 1 month, 3 months or 6 months under room temperature storage conditions (for example, 25°C).

In some embodiments, the present application discloses a pharmaceutical composition, when a packaged sample is stored in an accelerated condition (for example, $25 \pm 2°C$), an initial amount of aflibercept in the composition with a SEC-HPLC purity of $\geq 95\%$ remains unchanged after 1 month. In further embodiments, when the packaged sample is stored at a high temperature ($40 \pm 2°C$), the self-buffering high-concentration aflibercept pharmaceutical composition disclosed in the present application shows better effects than the high-concentration aflibercept pharmaceutical composition with a buffering system.

In some embodiments, the present application discloses a pharmaceutical composition, when insoluble particles with different particle sizes ($\geq 2\ \mu m$, $\geq 5\ \mu m$, $\geq 10\ \mu m$, and/or $\geq 25\ \mu m$) are measured, the self-buffering high-concentration aflibercept pharmaceutical composition disclosed in the present application shows better effects than the high-concentration aflibercept pharmaceutical composition with a buffering system.

In some embodiments, the present application discloses a self-buffering high-concentration aqueous or lyophilized pharmaceutical composition of the VEGF binding molecule, comprising: 1) a VEGF binding molecule, wherein the VEGF binding molecule is selected from the group consisting of a VEGF receptor fusion protein and an anti-VEGF antibody or an antigen-binding fragment thereof, and the concentration of the VEGF binding molecule is at least about 100 mg/mL; 2) a stabilizer, wherein the stabilizer is selected from the group consisting of sucrose, trehalose, proline, a pharmaceutically acceptable salt of proline, lysine, a pharmaceutically acceptable salt of lysine, sorbitol, and a combination thereof; 3) a surfactant, wherein the surfactant is selected from the group consisting of polysorbate 80, polysorbate 20, poloxamer, and a combination thereof; and wherein the aqueous pharmaceutical composition does not comprise a buffering agent and has a pH of about 5.0-7.0.

In some embodiments, the aqueous pharmaceutical composition does not comprise a buffering agent referred to a histidine buffering agent, a phosphate buffering agent, a citric acid buffering agent, an acetic acid buffering agent, a carbonic acid buffering agent, a succinic acid buffering agent, a tartaric acid buffering agent, and/or a maleic acid buffering agent.

In some embodiments, the self-buffering high-concentration aqueous or lyophilized pharmaceutical composition of a VEGF binding molecule further comprises: 4) at least one of L-arginine, a pharmaceutically acceptable salt of L-arginine, and sodium chloride.

In some embodiments, the concentration of L-arginine or the pharmaceutically acceptable salt of L-arginine is about 0-100 mM, or the concentration of sodium chloride is about 0-40 mM. In a preferred embodiment, the concentration of L-arginine or the pharmaceutically acceptable salt of L-arginine is about 20-100 mM, or the concentration of sodium chloride is about 40 mM. In a more preferred embodiment, the concentration of L-arginine or the pharmaceutically acceptable salt of L-arginine is about 20 mM, about 40 mM, about 50 mM, about 60 mM, about 70 mM, about 80 mM, or about 100 mM.

In some embodiments, the concentration of the VEGF binding molecule in the aqueous pharmaceutical composition is at least about 100 mg/mL, at least about 114.3 mg/mL, at least about 125 mg/mL, or at least about 180 mg/mL.

In some embodiments, the aqueous pharmaceutical composition comprises about 100-180 mg/mL of the VEGF binding molecule. In a preferred embodiment, the aqueous pharmaceutical composition comprises about 100-125 mg/mL of the VEGF binding molecule.

In some embodiments, the concentration of the stabilizer in the aqueous pharmaceutical composition is about 5-10% w/v. In some embodiments, the stabilizer is selected from the group consisting of about 5-9% w/v of sucrose and about 5.5-10% w/v of trehalose. In a preferred embodiment, the concentration of sucrose is about 5% w/v, about 6% w/v, about 7% w/v, about 8% w/v, or about 9% w/v, or the concentration of trehalose is about 5.5% w/v, about 6.6% w/v, about 7.7% w/v, about 8.0% w/v, or about 10% w/v.

In some embodiments, the surfactant is at least one selected from the group consisting of about 0.01-0.05% w/v of polysorbate 20, polysorbate 80, and poloxamer. In a preferred embodiment, the surfactant is polysorbate 20, and a concentration of polysorbate 20 is about 0.01% w/v, about 0.03% w/v, about 0.05% w/v, 0.07% w/v, about 0.1% w/v, or about 0.2% w/v.

In some embodiments, the pH of the aqueous pharmaceutical composition is about 5.5-6.4; preferably, the pH is about 5.5, about 5.8, about 6.0, about 6.1, about 6.2, or about 6.4.

In some embodiments, the aqueous pharmaceutical composition comprises a concentration of about 100-180 mg/mL of the VEGF receptor fusion protein, about 5-9% w/v of sucrose or about 5.5-10% w/v of trehalose, about 0.01-0.2% w/v of polysorbate 20, about 0-100 mM of L-arginine hydrochloride, or about 0-40 mM of sodium chloride; wherein the pH of the aqueous pharmaceutical composition is about 5.0-7.0.

In some embodiments, the aqueous pharmaceutical composition comprises a concentration of about 100-180 mg/mL of the VEGF receptor fusion protein, about 5-7% w/v of sucrose, or about 5.5-7.7% w/v of trehalose, about 0.01-0.2% w/v of polysorbate 20, about 20-100 mM of L-arginine hydrochloride, or about 0-40 mM of sodium chloride; wherein the pH of the aqueous pharmaceutical composition is about 5.5-6.4.

In some embodiments, the aqueous pharmaceutical composition is any one selected from the group consisting of the followings:

an aqueous pharmaceutical composition A comprising a concentration of about 114.3 mg/mL of the VEGF receptor fusion protein, about 5% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition B comprising a concentration of about 114.3 mg/mL of the VEGF receptor fusion protein, about 6% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition C comprising a concentration of about 114.3 mg/mL of the VEGF receptor fusion protein, about 7% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition D comprising a concentration of about 114.3 mg/mL of the VEGF receptor fusion protein, about 5.5% w/v of trehalose, about 0.03% w/v of polysorbate 20 and about 50 mM of L-arginine hydrochloride, wherein the pH of the aqueous pharmaceutical composition is about 5.5-6.4;

an aqueous pharmaceutical composition E comprising a concentration of about 114.3 mg/mL of the VEGF receptor fusion protein, about 6.6% w/v of trehalose, about 0.03% w/v of polysorbate 20, and about 50 mM of L-arginine hydrochloride, wherein the pH of the aqueous pharmaceutical composition is about 5.5-6.4;

an aqueous pharmaceutical composition F comprising a concentration of about 114.3 mg/mL of the VEGF receptor fusion protein, about 7.7% w/v of trehalose, about 0.03% w/v of polysorbate 20, and about 50 mM of L-arginine hydrochloride, wherein the pH of the aqueous pharmaceutical composition is about 5.5-6.4;

an aqueous pharmaceutical composition G comprising a concentration of about 114.3 mg/mL of the VEGF receptor fusion protein, about 5% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition H comprising a concentration of about 114.3 mg/mL of the VEGF receptor fusion protein, about 6% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition I comprising a concentration of about 114.3 mg/mL of the VEGF receptor fusion protein, about 7% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition J comprising a concentration of about 114.3 mg/mL of the VEGF receptor fusion protein, about 5.5% w/v of trehalose, about 0.03% w/v of polysorbate 20, and about 60 mM of L-arginine hydrochloride, wherein the pH of the aqueous pharmaceutical composition is about 5.5-6.4;

an aqueous pharmaceutical composition K comprising a concentration of about 114.3 mg/mL of the VEGF receptor fusion protein, about 6.6% w/v of trehalose, about 0.03% w/v of polysorbate 20, and about 60 mM of L-arginine hydrochloride, wherein the pH of the aqueous pharmaceutical composition is about 5.5-6.4;

an aqueous pharmaceutical composition L comprising a concentration of about 114.3 mg/mL of the VEGF receptor fusion protein, about 7.7% w/v of trehalose, about 0.03% w/v of polysorbate 20, and about 60 mM of L-arginine hydrochloride, wherein the pH of the aqueous pharmaceutical composition is about 5.5-6.4;

an aqueous pharmaceutical composition M comprising a concentration of about 114.3 mg/mL of the VEGF receptor fusion protein, about 5% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition N comprising a concentration of about 114.3 mg/mL of the VEGF receptor fusion protein, about 6% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition O comprising a concentration of about 114.3 mg/mL of the VEGF receptor fusion protein, about 7% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition P comprising a concentration of about 114.3 mg/mL of the VEGF receptor fusion protein, about 5.5% w/v of trehalose, about 0.03% w/v of polysorbate 20, and about 70 mM of L-arginine hydrochloride, wherein the pH of the aqueous pharmaceutical composition is about 5.5-6.4;

an aqueous pharmaceutical composition Q comprising a concentration of about 114.3 mg/mL of the VEGF receptor fusion protein, about 6.6% w/v of trehalose, about 0.03% w/v of polysorbate 20, and about 70 mM of L-arginine hydrochloride, wherein the pH of the aqueous pharmaceutical composition is about 5.5-6.4;

an aqueous pharmaceutical composition R comprising a concentration of about 114.3 mg/mL of the VEGF receptor fusion protein, about 7.7% w/v of trehalose, about 0.03% w/v of polysorbate 20, and about 70 mM of L-arginine hydrochloride, wherein the pH of the aqueous pharmaceutical composition is about 5.5-6.4;

an aqueous pharmaceutical composition S comprising a concentration of about 114.3 mg/mL of the VEGF receptor fusion protein, about 5% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition T comprising a concentration of about 114.3 mg/mL of the VEGF receptor fusion protein, about 6% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition U comprising a concentration of about 114.3 mg/mL of the VEGF receptor fusion protein, about 7% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition V comprising a concentration of about 114.3 mg/mL of the VEGF receptor fusion protein, about 5.5% w/v of trehalose, about 0.03% w/v of polysorbate 20, and about 80 mM of L-arginine hydrochloride, wherein the pH of the aqueous pharmaceutical composition is about 5.5-6.4;

an aqueous pharmaceutical composition W comprising a concentration of about 114.3 mg/mL of the VEGF receptor fusion protein, about 6.6% w/v of trehalose, about 0.03% w/v of polysorbate 20, and about 80 mM of L-arginine hydrochloride, wherein the pH of the aqueous pharmaceutical composition is about 5.5-6.4;

an aqueous pharmaceutical composition X comprising a concentration of about 114.3 mg/mL of the VEGF receptor fusion protein, about 7.7% w/v of trehalose, about 0.03% w/v of polysorbate 20, and about 80 mM of L-arginine hydrochloride, wherein the pH of the aqueous pharmaceutical composition is about 5.5-6.4;

an aqueous pharmaceutical composition Y comprising a concentration of about 114.3 mg/mL of the VEGF receptor fusion protein, about 5% w/v of sucrose, about 0.01% w/v of polysorbate 20, and about 50 mM of L-arginine hydrochloride, wherein the pH of the aqueous pharmaceutical composition is about 5.5-6.4;

an aqueous pharmaceutical composition Z comprising a concentration of about 114.3 mg/mL of the VEGF receptor fusion protein, about 5% w/v of sucrose, about 0.05% w/v of polysorbate 20, and about 50 mM of L-arginine hydrochloride, wherein the pH of the aqueous pharmaceutical composition is about 5.5-6.4;

an aqueous pharmaceutical composition ZA comprising a concentration of about 114.3 mg/mL of the VEGF receptor fusion protein, about 5% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition ZB comprising a concentration of about 125 mg/mL of the VEGF receptor fusion protein, about 6% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition ZC comprising a concentration of about 125 mg/mL of the VEGF receptor fusion protein, about 7% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition ZD comprising a concentration of about 125 mg/mL of the VEGF receptor fusion protein, about 5.5% w/v of trehalose, about 0.03% w/v of polysorbate 20 and about 50 mM of L-arginine hydrochloride, wherein the pH of the aqueous pharmaceutical composition is about 5.5-6.4;

an aqueous pharmaceutical composition ZE comprising a concentration of about 125 mg/mL of the VEGF receptor fusion protein, about 6.6% w/v of trehalose, about 0.03% w/v of polysorbate 20, and about 50 mM of L-arginine hydrochloride, wherein the pH of the aqueous pharmaceutical composition is about 5.5-6.4;

an aqueous pharmaceutical composition ZF comprising a concentration of about 125 mg/mL of the VEGF receptor fusion protein, about 7.7% w/v of trehalose, about 0.03% w/v of polysorbate 20, and about 50 mM of L-arginine hydrochloride, wherein the pH of the aqueous pharmaceutical composition is about 5.5-6.4;

an aqueous pharmaceutical composition ZG comprising a concentration of about 125 mg/mL of the VEGF receptor fusion protein, about 5% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition ZH comprising a concentration of about 125 mg/mL of the VEGF receptor fusion protein, about 6% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition ZI comprising a concentration of about 125 mg/mL of the VEGF receptor fusion protein, about 7% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition ZJ comprising a concentration of about 125 mg/mL of the VEGF receptor fusion protein, about 5.5% w/v of trehalose, about 0.03% w/v of polysorbate 20 and about 60 mM of L-arginine hydrochloride, wherein the pH of the aqueous pharmaceutical composition is about 5.5-6.4;

an aqueous pharmaceutical composition ZK comprising a concentration of about 125 mg/mL of the VEGF receptor fusion protein, about 6.6% w/v of trehalose, about 0.03% w/v of polysorbate 20, and about 60 mM of L-arginine hydrochloride, wherein the pH of the aqueous pharmaceutical composition is about 5.5-6.4;

an aqueous pharmaceutical composition ZL comprising a concentration of about 125 mg/mL of the VEGF receptor fusion protein, about 7.7% w/v of trehalose, about 0.03% w/v of polysorbate 20, and about 60 mM of L-arginine hydrochloride, wherein the pH of the aqueous pharmaceutical composition is about 5.5-6.4;

an aqueous pharmaceutical composition ZM comprising a concentration of about 125 mg/mL of the VEGF receptor fusion protein, about 5% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition ZN comprising a concentration of about 125 mg/mL of the VEGF receptor fusion protein, about 6% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition ZO comprising a concentration of about 125 mg/mL of the VEGF receptor fusion protein, about 7% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition ZP comprising a concentration of about 125 mg/mL of the VEGF receptor fusion protein, about 5.5% w/v of trehalose, about 0.03% w/v of polysorbate 20 and about 70 mM of L-arginine hydrochloride, wherein the pH of the aqueous pharmaceutical composition is about 5.5-6.4;

an aqueous pharmaceutical composition ZQ comprising a concentration of about 125 mg/mL of the VEGF receptor fusion protein, about 6.6% w/v of trehalose, about 0.03% w/v of polysorbate 20, and about 70 mM of L-arginine hydrochloride, wherein the pH of the aqueous pharmaceutical composition is about 5.5-6.4;

an aqueous pharmaceutical composition ZR comprising a concentration of about 125 mg/mL of the VEGF receptor fusion protein, about 7.7% w/v of trehalose, about 0.03% w/v of polysorbate 20, and about 70 mM of L-arginine hydrochloride, wherein the pH of the aqueous pharmaceutical composition is about 5.5-6.4;

an aqueous pharmaceutical composition ZS comprising a concentration of about 125 mg/mL of the VEGF receptor fusion protein, about 5% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition ZT comprising a concentration of about 125 mg/mL of the VEGF receptor fusion protein, about 6% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition ZU comprising a concentration of about 125 mg/mL of the VEGF receptor fusion protein, about 7% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition ZV comprising a concentration of about 125 mg/mL of the VEGF receptor fusion protein, about 5.5% w/v of trehalose, about 0.03% w/v of polysorbate 20 and about 80 mM of L-arginine hydrochloride, wherein the pH of the aqueous pharmaceutical composition is about 5.5-6.4;

an aqueous pharmaceutical composition ZW comprising a concentration of about 125 mg/mL of the VEGF receptor fusion protein, about 6.6% w/v of trehalose, about 0.03% w/v of polysorbate 20, and about 80 mM of L-arginine hydrochloride, wherein the pH of the aqueous pharmaceutical composition is about 5.5-6.4;

an aqueous pharmaceutical composition ZX comprising a concentration of about 125 mg/mL of the VEGF receptor fusion protein, about 7.7% w/v of trehalose, about 0.03% w/v of polysorbate 20, and about 80 mM of L-arginine hydrochloride, wherein the pH of the aqueous pharmaceutical composition is about 5.5-6.4;

an aqueous pharmaceutical composition ZY comprising a concentration of about 125 mg/mL of the VEGF receptor fusion protein, about 5% w/v of sucrose, about 0.01% w/v of polysorbate 20, and about 50 mM of L-arginine hydrochloride, wherein the pH of the aqueous pharmaceutical composition is about 5.5-6.4;

an aqueous pharmaceutical composition ZZ comprising a concentration of about 125 mg/mL of the VEGF receptor fusion protein, about 5% w/v of sucrose, about 0.05% w/v of polysorbate 20, and about 50 mM of L-arginine hydrochloride, wherein the pH of the aqueous pharmaceutical composition is about 5.5-6.4;

an aqueous pharmaceutical composition AA comprising a concentration of about 180 mg/mL of the VEGF receptor fusion protein, about 5% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition AB comprising a concentration of about 180 mg/mL of the VEGF receptor fusion protein, about 6% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition AC comprising a concentration of about 180 mg/mL of the VEGF receptor fusion protein, about 7% w/v of sucrose, about 0.03% w/v of polysorbate 20, and about 50 mM of L-arginine hydrochloride, wherein the pH of the aqueous pharmaceutical composition is about 5.5-6.4;

an aqueous pharmaceutical composition AD comprising a concentration of about 180 mg/mL of the VEGF receptor fusion protein, about 5.5% w/v of trehalose, about 0.03% w/v of polysorbate 20 and about 50 mM of L-arginine hydrochloride, wherein the pH of the aqueous pharmaceutical composition is about 5.5-6.4;

an aqueous pharmaceutical composition AE comprising a concentration of about 180 mg/mL of the VEGF receptor fusion protein, about 6.6% w/v of trehalose, about 0.03% w/v of polysorbate 20, and about 50 mM of L-arginine hydrochloride, wherein the pH of the aqueous pharmaceutical composition is about 5.5-6.4;

an aqueous pharmaceutical composition AF comprising a concentration of about 180 mg/mL of the VEGF receptor fusion protein, about 7.7% w/v of trehalose, about 0.03% w/v of polysorbate 20, and about 50 mM of L-arginine hydrochloride, wherein the pH of the aqueous pharmaceutical composition is about 5.5-6.4;

an aqueous pharmaceutical composition AG comprising a concentration of about 180 mg/mL of the VEGF receptor fusion protein, about 5% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition AH comprising a concentration of about 180 mg/mL of the VEGF receptor fusion protein, about 6% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition AI comprising a concentration of about 180 mg/mL of the VEGF receptor fusion protein, about 7% w/v of sucrose, about 0.03% w/v of polysorbate 20, and about 60 mM of L-arginine hydrochloride, wherein the pH of the aqueous pharmaceutical composition is about 5.5-6.4;

an aqueous pharmaceutical composition AJ comprising a concentration of about 180 mg/mL of the VEGF receptor fusion protein, about 5.5% w/v of trehalose, about 0.03% w/v of polysorbate 20 and about 60 mM of L-arginine hydrochloride, wherein the pH of the aqueous pharmaceutical composition is about 5.5-6.4;

an aqueous pharmaceutical composition AK comprising a concentration of about 180 mg/mL of the VEGF receptor fusion protein, about 6.6% w/v of trehalose, about 0.03% w/v of polysorbate 20, and about 60 mM of L-arginine hydrochloride, wherein the pH of the aqueous pharmaceutical composition is about 5.5-6.4;

an aqueous pharmaceutical composition AL comprising a concentration of about 180 mg/mL of the VEGF receptor fusion protein, about 7.7% w/v of trehalose, about 0.03% w/v of polysorbate 20, and about 60 mM of L-arginine hydrochloride, wherein the pH of the aqueous pharmaceutical composition is about 5.5-6.4;

an aqueous pharmaceutical composition AM comprising a concentration of about 180 mg/mL of the VEGF receptor fusion protein, about 5% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition AN comprising a concentration of about 180 mg/mL of the VEGF receptor fusion protein, about 6% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition AO comprising a concentration of about 180 mg/mL of the VEGF receptor fusion protein, about 7% w/v of sucrose, about 0.03% w/v of polysorbate 20, and about 70 mM of L-arginine hydrochloride, wherein the pH of the aqueous pharmaceutical composition is about 5.5-6.4;

an aqueous pharmaceutical composition AP comprising a concentration of about 180 mg/mL of the VEGF receptor fusion protein, about 5.5% w/v of trehalose, about 0.03% w/v of polysorbate 20 and about 70 mM of L-arginine hydrochloride, wherein the pH of the aqueous pharmaceutical composition is about 5.5-6.4;

an aqueous pharmaceutical composition AQ comprising a concentration of about 180 mg/mL of the VEGF receptor fusion protein, about 6.6% w/v of trehalose, about 0.03% w/v of polysorbate 20, and about 70 mM of L-arginine hydrochloride, wherein the pH of the aqueous pharmaceutical composition is about 5.5-6.4;

an aqueous pharmaceutical composition AR comprising a concentration of about 180 mg/mL of the VEGF receptor fusion protein, about 7.7% w/v of trehalose, about 0.03% w/v of polysorbate 20, and about 70 mM of L-arginine hydrochloride, wherein the pH of the aqueous pharmaceutical composition is about 5.5-6.4;

an aqueous pharmaceutical composition AS comprising a concentration of about 180 mg/mL of the VEGF receptor fusion protein, about 5% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition AT comprising a concentration of about 180 mg/mL of the VEGF receptor fusion protein, about 6% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition AU comprising a concentration of about 180 mg/mL of the VEGF receptor fusion protein, about 7% w/v of sucrose, about 0.03% w/v of polysorbate 20, and about 80 mM of L-arginine hydrochloride, wherein the pH of the aqueous pharmaceutical composition is about 5.5-6.4;

an aqueous pharmaceutical composition AV comprising a concentration of about 180 mg/mL of the VEGF receptor fusion protein, about 5.5% w/v of trehalose, about 0.03% w/v of polysorbate 20 and about 80 mM of L-arginine hydrochloride, wherein the pH of the aqueous pharmaceutical composition is about 5.5-6.4;

an aqueous pharmaceutical composition AW comprising a concentration of about 180 mg/mL of the VEGF receptor fusion protein, about 6.6% w/v of trehalose, about 0.03% w/v of polysorbate 20, and about 80 mM of L-arginine hydrochloride, wherein the pH of the aqueous pharmaceutical composition is about 5.5-6.4;

an aqueous pharmaceutical composition AX comprising a concentration of about 180 mg/mL of the VEGF receptor fusion protein, about 7.7% w/v of trehalose, about 0.03% w/v of polysorbate 20, and about 80 mM of L-arginine hydrochloride, wherein the pH of the aqueous pharmaceutical composition is about 5.5-6.4;

an aqueous pharmaceutical composition AY comprising a concentration of about 180 mg/mL of the VEGF receptor fusion protein, about 5% w/v of sucrose, about 0.01% w/v of polysorbate 20, and about 50 mM of L-arginine hydrochloride, wherein the pH of the aqueous pharmaceutical composition is about 5.5-6.4;

an aqueous pharmaceutical composition AZ comprising a concentration of about 180 mg/mL of the VEGF receptor fusion protein, about 5% w/v of sucrose, about 0.05% w/v of polysorbate 20, and about 50 mM of L-arginine hydrochloride, wherein the pH of the aqueous pharmaceutical composition is about 5.5-6.4; and

an aqueous pharmaceutical composition BA comprising a concentration of about 114.3 mg/mL of the VEGF receptor fusion protein, about 8% w/v of trehalose, about 0.03% w/v of polysorbate 20, and about 20 mM of L-arginine hydrochloride, wherein the pH of the aqueous pharmaceutical composition is about 5.5-6.4;

preferably, the VEGF receptor fusion protein is aflibercept.

In some embodiments, the VEGF binding molecule comprises two polypeptides, each of the polypeptides comprises an immunoglobulin-like (Ig) domain 2 of VEGFR1 and an immunoglobulin-like (Ig) domain 3 of VEGFR2. In a preferred embodiment, the VEGF binding molecule is selected from the group consisting of bevacizumab, ranibizumab, aflibercept, and conbercept. In a more preferred embodiment, the VEGF binding molecule is aflibercept.

In some embodiments, the pharmaceutical composition is stable at 2-8°C for at least 6 months, 12 months, or 24 months; or the pharmaceutical composition is stable at 25 ± 2°C for at least 1 month, 3 months or 6 months.

In some embodiments, the present application provides a lyophilized formulation, wherein the lyophilized formulation is obtained by lyophilizing the aqueous pharmaceutical composition according to the present application, or the lyophilized formulation is reconstituted to obtain the aqueous pharmaceutical composition of the present application.

In some embodiments, the present application provides a delivery device comprising the aqueous pharmaceutical composition or the lyophilized formulation after reconstitution according to the present application. In one preferred embodiment, the delivery device is a pre-filled syringe.

In some embodiments, the present application provides a method for treating an ocular disease or disorder mediated by VEGF comprising administering the aqueous pharmaceutical composition or delivery device according to the present application to a subject. In one preferred embodiment, a site of administration is intravitreal.

In some embodiments, the present application provides use of the aqueous pharmaceutical composition or delivery device according to the present application in the manufacture of a pharmaceutical composition for the treatment of an ocular disease or disorder mediated by VEGF, comprising administering the aqueous pharmaceutical composition or delivery device according to the present application to a subject. In a preferred embodiment, a site of administration is intravitreal.

In some embodiments, the ocular disease or disorder comprises an ocular neovascular disease or an ocular vascular disease.

In some embodiments, the ocular disease or disorder comprises neovascular (wet) senile macular degeneration (AMD), age-related macular degeneration (wet), macular edema, macular edema after retinal vein occlusion, retinal vein occlusion (RVO), central retinal vein occlusion (CRVO), branch retinal vein occlusion (BRVO), diabetic macular edema (DME), choroidal neovascularization (CNV), iris neovascularization, neovascular glaucoma, postoperative fibrosis of glaucoma, proliferative vitreoretinopathy (PVR), optic disc neovascularization, corneal neovascularization, retinal neovascularization, vitreous neovascularization, corneal pannus, pterygium, vascular retinopathy, diabetic retinopathy (DR), non-proliferative diabetic retinopathy, and/or proliferative diabetic retinopathy.

In some embodiments, a method for delivering a VEGF binding molecule to a subject is disclosed, comprising a step of administering the aqueous pharmaceutical composition or delivery device according to the present application to the subject. In one preferred embodiment, a site of administering is vitreous body.

The pharmaceutical composition provided in the present application does not need an additional buffering agent, and has high stability, convenient storage and wide application prospects.

## BRIEF DESCRIPTION OF THE DRAWINGS

The drawings further illustrate the novel features disclosed in the present application. The features and advantages disclosed in the present application will be better understood by referring to these drawings, but it should be understood that these drawings are only used to illustrate specific embodiments of the principles disclosed in the present application and are not intended to limit the scope of the appended claims.

FIG. 1 is a trend diagram of SEC main peak purity transformation of formulations A2 and A3 at 40 ± 2°C.

FIG. 2 is a trend diagram of SEC polymer purity transformation of formulations A2 and A3 under different storage conditions.

FIG. 3 is a trend diagram of SEC main peak purity transformation of formulations B1, B2, B3, B4, and B5 under different storage conditions.

FIG. 4 is a curve graph of viscosities corresponding to formulations B1, B2, B3, B4, and B5.

FIG. 5 is a trend diagram of SEC main peak purity transformation of formulations C1-C10 at 37 ± 2°C.

FIG. 6 is a trend diagram of SEC purity transformation of formulations F5 and F6 at 2-8°C and 37 ± 2°C.

## DETAILED DESCRIPTION

The term "about" includes and describes the value or parameter itself. For example, "about x" includes and describes "x" itself. As used in the present application, the term "about", when used in combination with a measured value or to modify a value, unit, constant or series of values, refers to a variation ranging from ± 1% to ± 10% in addition to the value or parameter itself. In some embodiments, the term "about" refers to a variation of ± 1%, ± 2%, ± 3%, ± 4%, ± 5%, ± 6%, ± 7%, ± 8%, ± 9%, or ± 10% when used in combination with a measured value or to modify a value, unit, constant or a series of values.

The term "aqueous" pharmaceutical composition refers to a pharmaceutical composition comprising water.

The term "self-buffering" refers to the exclusion of components traditionally used to introduce a buffering capacity into formulations, for example, the exclusion buffers include but are not limited to histidine buffering agent, phosphate buffering agent, citric acid buffering agent, acetic acid buffering agent, carbonic acid buffering agent, succinic acid buffering agent, tartaric acid buffering agent, maleic acid buffering agent or mixtures thereof, and the like. In addition to any buffering capacity present in a formulation comprising no buffer components, protein is known to provide buffering capacity for the formulation. Therefore, these terms do not mean that the formula cannot have any buffering capacity.

The term "high-concentration" formulation comprises a concentration of VEGF receptor fusion protein with a concentration of at least 100, 101, 102, 103, 104, 105, 106, 107, 108, 109, 110, 111, 112, 113, 114, 114.1, 114.2, 114.3, 114.4, 114.5, 114.6, 114.7, 114.8, 114.9, 115, 116, 117, 118, 119, 120, 121, 122, 123, 124, 125, 126, 127, 128, 129, 130, 131, 132, 133, 134, 135, 136, 137, 138, 139, 140, 141,142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, and 180 mg/mL.

The term "VEGF binding molecule" refers to a VEGF receptor fusion protein, an anti-VEGF antibody or an antigen-binding fragment or an antibody or a fragment that specifically binds to VEGF. An exemplary VEGF receptor fusion protein includes aflibercept (Regeneron Pharmaceuticals, Inc.), see International Patent Application Publication WO2005/121176 or WO2007/112675.

The term "VEGF receptor fusion protein" refers to a molecule comprising one or more VEGF receptors or domains thereof fused to another polypeptide, which interferes with the interaction between VEGF and natural VEGF receptors. For example, two such fusion polypeptides combine to form a homodimer or other polymer. Such a VEGF receptor fusion protein can be called "VEGF-Trap" or "VEGF Trap". The VEGF receptor fusion protein falling within this definition within the scope of the present application includes a chimeric polypeptide, which contains two or more immunoglobulin (Ig)-like domains of the VEGF receptor, such as VEGFR1 (also called Flt1) and/or VEGFR2 (also called Flk1 or KDR), and may also contain a polydomain (such as an Fc domain).

The term "aflibercept" comprises a biosimilar form thereof, which generally refers to a product comprising the same amino acid sequence. The amino acid sequence and nucleic acid sequence of aflibercept in the examples have been recited in the literature, as shown in WO2000075319A1.

The term "treatment" as used herein refers to a treatment mean described in the present application. A method of the "treatment" adopts administering the antibody of the present application to a subject in need of such treatment (for example, a subject with a VEGF-mediated ocular disorder or a subject who may eventually acquire such disorder) to prevent, cure, delay the disorder or recurrent disorder, reduce the severity of the disorder or recurrent disorder, or improve one or more symptoms of the disorder or recurrent disorder, or to prolong the survival of the subject beyond what would be expected without such treatment.

The term "subject" or "patient" refers to a human and a non-human mammal, including but not limited to a primate, rabbit, pig, horse, dog, cat, sheep and cattle.

Determination of protein concentration: The protein concentration is determined by Lunatic micro-spectrum analyzer. The blank is deducted with purified water, the ultraviolet absorption of 280 nm is corrected by light scattering of 330 nm, and the protein concentration is calculated according to Formula 1, wherein the extinction coefficient $\varepsilon$ is 1.5 (L/g cm$^{-1}$).

$$\text{Protein concentration} = \frac{A(280) - A(330)}{\varepsilon}$$

Formula 1

Size Exclusion Chromatography (SEC): Size exclusion chromatography is used to quantify polymers, monomers, and fragments. The determination is carried out on a TSKgel G3000SWXL 7.8×300 mm 5 µm 250Å column and run on Waters e2695-2489 HPLC system. The mobile phase is a potassium phosphate buffering agent. The sample is diluted with the mobile phase to 1 mg/mL, and the injection volume is 25 µL. The protein is eluted isotropically at a flow rate of 0.5 mL/min for 30 min, and the absorbance of the eluate is detected at 215 nm. Empower 3 software is used for integration processing.

Reduced capillary electrophoresis (rCE-SDS): The purity % of (main peak 1+ main peak 2) was determined by reduced capillary electrophoresis (rCE-SDS), and this determination is carried out on a SCIEX PA800 plus capillary electrophoresis system with an uncoated quartz capillary of 50 µm I.D. The effective separation length of the capillary is 20 cm, the total length is 30.2 cm, and the bandwidth of PDA 220 nm is 10 nm. 32Karat software is used for integration processing.

Non-reduced SDS-PAGE (nrSDS-PAGE): The purity % of main peak is determined by non-reduced SDS-PAGE electrophoresis. This determination is carried out by polyacrylamide gel electrophoresis. The concentration of separation gel is 8%, and the concentration of concentrating gel is 4.5%. The electrophoresis is carried out at a constant current of 10 mA/gel. After staining with Coomassie Brilliant Blue R250 and decolorizing with a decoloring solution, it is scanned and imaged by a gel imaging system, and the purity of the target band is analyzed.

Imaging capillary isoelectric focusing electrophoresis (iCIEF): The charge heterogeneity and identification experiment are determined by the imaging capillary isoelectric focusing electrophoresis (iCIEF) method. This determination is carried out on the Maurice C system of the imaging capillary electrophoresis instrument. The effective separation length of the capillary is 5 cm, the focusing time is 6 min, and the focusing program is 1.5kV-1min and 3kV-5min. Empower 3 software is used for integration processing.

MFI Microfluidic Imaging Particle Analysis: The number of particles is determined by MFI Microfluidic Imaging Particle Analysis System, and this determination is carried out in MFI Particle Analysis System MFI 5200. The volume of the sample to be tested is 1 mL, the total effective volume is 0.7 mL, and the analysis volume is 0.51 mL. MVSS analysis software is used for analysis.

Determination of viscosity by m-VROC viscometer: e-VROC™ Constructed by VROC® technology of Rheosense is an excellent tool for measuring both extensional viscosity and shear viscosity simultaneously. The viscometer uses MEMS pressure sensors to measure the pressures upstream and downstream of contraction, and records the flow changes. The fluid undergoes almost constant extension through contraction/expansion, so that the apparent extensional viscosity can be calculated.

Determination of osmolality by freezing point depression: Osmolality is indirectly determined by measuring the freezing point depression of the solution. In a desired dilute solution, the freezing point depression follows the relationship of $\Delta Tf = Kf \cdot m$, wherein $\Delta Tf$ is the freezing point depression, Kf is the freezing point depression constant (=1.86 when water is the solvent), and m is the weight molar concentration. The osmotic pressure conforms to the relationship of $Po = ko \cdot m$, wherein Po is the osmotic pressure, ko is the osmotic pressure constant, and m is the weight molar concentration of the solution. The concentrations in the two formulas are equivalent, so the osmolality of the solution can be determined by freezing point depression.

Determination of binding activity by ELISA: The binding activity of recombinant human vascular endothelial growth factor receptor-antibody fusion protein injection with VEGF165 is determined by ELISA. Firstly, the human VEGF165 protein is coated on a 96-well plate, then the gradient diluted product is added, and then the secondary antibody, chromogenic solution and stop solution are added in turn, and the absorbance value is read to calculate the binding ability of the product with human VEGF165 antigen. The test data are analyzed by four-parameter fitting with Soft Max Pro or other similar software, and the results are automatically analyzed according to the following formula: the logarithmic value of the concentration of the reference substance (self-made) is the X axis, and the absorbance value is the Y axis. The software will give the half effective concentration ($EC_{50}$) of the test substance and the reference substance, and draw an "S" curve.

$$\text{Binding activity}(\%) = \frac{\text{Reference } EC_{50} \, (\mu g/mL)}{\text{Test } EC_{50} \, (\mu g/mL)} \times 100\%$$

The concentrations of the VEGF binding molecule, the stabilizer, the surfactant, L-arginine, the pharmaceutically acceptable salt of L-arginine and sodium chloride mentioned in the present application refer to the concentrations of the VEGF binding molecule, the stabilizer, the surfactant, L-arginine, the pharmaceutically acceptable salt of L-arginine and sodium chloride in the aqueous pharmaceutical composition respectively.

The experimental conditions in the following examples are: oscillating at room temperature (25 ± 2°C) for one week (25°C 1 W), three freeze-thaw cycles (freezing at -40°C for 16 h and melting at 25°C for 8 h as a cycle), 2-8°C for 1 month (2-8°C 1 M), 2-8°C for 6 months (2-8°C 6 M), 25 ± 2°C for 1 month (25°C 1 M), 40 ± 2°C for one week (40°C 1 W), 40°C ± 2°C for two weeks (40°C 2 W) or 40 ± 2°C for one month (40°C 1 M).

**Example 1: Preparation of a self-buffering system formulation sample**

The long-term efficacy, physical stability and the formation of charge heterosomes of four different formulations with VEGF receptor fusion proteins (aflibercept) of about 40 mg/mL, about 114.3 mg/mL and about 180 mg/mL were tested. The component concentrations of the four formulations were shown in Table 1.1. In order to prepare the formulation solution, the buffering agent was exchange with the expected adjuvant solution and concentrated to the desired antibody concentration by ultrafiltration. After the ultrafiltration concentration was completed, the adjuvant to be added was added to the antibody solution in the form of mother liquor. All the formulations were aseptically filtered through a 0.22 μm low protein binding filter, and filled into a 2 mL sterile vial under aseptic conditions, and sealed with a film coated rubber stopper and a cap made of aluminum-plastics combinations. SEC-HPLC, nr-SDS-PAGE and rCE-SDS were used to determine the presence (%) of high molecular weight species (HMWS, LMWS) and main peaks (Monomer/MAIN), and to evaluate the stability of aflibercept of each formulation during storage under different conditions.

The following are different formulations and their stability results:

Table 1.1 Composition of formulations comprising aflibercept

| Test Examples | Aflibercept (mg/mL) | Histidine (mM) | Sodium phosphate (mM) | Sucrose (% w/v) | Polysorbate20 (% w/v) | Sodium chloride (mM) | L-arginine hydrochloride (mM) | pH |
|---|---|---|---|---|---|---|---|---|
| A1 | 40 | - | 10 | 5 | 0.03 | 40 | - | 6.2 |
| A2 | 114.3 | 10 | - | 5 | 0.03 | - | 50 | 5.8 |
| A3 | 114.3 | - | - | 6 | 0.03 | - | 50 | 5.8 |
| A4 | 180 | - | - | 6 | 0.03 | - | 50 | 5.8 |
| Note: "-" means not added. | | | | | | | | |

Table 1.2 Stability data of formulation A1

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.89 | 99.1 | / | 1.9 | 96.9 | 1.2 | / | 97.9 | 2.1 |
| Oscillating 1W | 1 | 99 | / | 1.3 | 96.8 | 1.8 | / | 97.7 | 2.3 |
| Three freeze-thaw cycles | 1.1 | 98.9 | / | 1.4 | 96.8 | 1.7 | / | 97.8 | 2.2 |
| 2-8°C 1M | 1.2 | 98.8 | / | 2.9 | 95.6 | 1.2 | 0.09 | 97.9 | 2.1 |
| 2-8°C 6M | 1.1 | 98.9 | / | 2.4 | 95.5 | 2.1 | / | 97.7 | 2.3 |
| 25°C 1M | 1.5 | 98.5 | / | 3.4 | 93.9 | 2.6 | 0.09 | 97.3 | 2.6 |
| 40°C 1W | 4.8 | 95.1 | 0.13 | 1.8 | 95.0 | 3.1 | / | 96.7 | 3.3 |
| 40°C 2W | 7 | 92.9 | 0.1 | 2.6 | 93.1 | 4.2 | 0.26 | 95.8 | 3.9 |
| Note: "/" means not detected. | | | | | | | | | |

Table 1.3 Stability data of formulation A2

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.89 | 99.1 | / | 1.8 | 96.9 | 1.2 | / | 98.2 | 1.8 |
| Oscillating 1W | 1.1 | 98.9 | / | 1.7 | 96.4 | 1.8 | / | 97.8 | 2.3 |
| Three freeze-thaw cycles | 1.1 | 98.9 | / | 1.2 | 96.9 | 1.9 | / | 97.7 | 2.4 |
| 2-8°C 1M | 1.2 | 98.8 | / | 2.8 | 95.6 | 1.4 | 0.05 | 97.9 | 2.1 |
| 2-8°C 6M | 1.1 | 98.9 | / | 2.2 | 95.4 | 2.3 | / | 97.6 | 2.4 |
| 25°C 1M | 1.6 | 98.4 | / | 3.3 | 93.5 | 3.0 | 0.08 | 97.3 | 2.6 |
| 40°C 1W | 16.5 | 83.4 | 0.15 | 1.9 | 93.8 | 4.2 | / | 96.2 | 3.8 |
| 40°C 2W | 22.8 | 77.1 | 0.11 | 3.2 | 90.9 | 6.0 | 0.24 | 95.6 | 4.2 |
| 40°C 1M | 41 | 58.8 | 0.25 | 8.9 | 79.4 | 11.6 | 0.12 | 93.3 | 6.6 |
| Note: "/" means not detected. | | | | | | | | | |

Table 1.4 Stability data of formulation A3

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.1 | 98.9 | / | 2.0 | 96.7 | 1.2 | / | 98.2 | 1.8 |
| Oscillating 1W | 1.4 | 98.6 | / | 2.0 | 96.1 | 1.8 | / | 97.7 | 2.4 |
| Three freeze-thaw cycles | 1.3 | 98.7 | / | 1.5 | 96.5 | 2.0 | / | 97.7 | 2.3 |
| 2-8°C 1M | 1.5 | 98.5 | / | 3.2 | 95.3 | 1.3 | 0.10 | 97.8 | 2.1 |
| 2-8°C 6M | 1.3 | 98.7 | / | 2.7 | 95.0 | 2.3 | / | 97.8 | 2.2 |
| 25°C 1M | 2 | 98 | / | 4.1 | 93.4 | 2.5 | 0.09 | 97.4 | 2.5 |
| 40°C 1W | 13.5 | 86.4 | 0.13 | 2.3 | 94.4 | 3.3 | / | 96.7 | 3.3 |
| 40°C 2W | 18.3 | 81.6 | 0.18 | 3.5 | 92.2 | 4.2 | 0.14 | 96.0 | 3.9 |
| 40°C 1M | 35.1 | 64.7 | 0.25 | 9.6 | 80.8 | 9.7 | 0.14 | 93.9 | 5.9 |
| Note: "/"means not detected. | | | | | | | | | |

Table 1.5 Stability data of formulation A4

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.2 | 98.8 | / | 2.3 | 96.5 | 1.2 | / | 98.1 | 1.9 |
| Three freeze-thaw cycles | 1.5 | 98.5 | / | 1.9 | 96.4 | 1.5 | / | 97.7 | 2.3 |
| 2-8°C 1M | 1.8 | 98.2 | / | 3.5 | 95.2 | 1.2 | 0.10 | 97.7 | 2.2 |
| 2-8°C 6M | 1.6 | 98.4 | / | 3.1 | 94.6 | 2.3 | / | 97.4 | 2.6 |
| 25°C 1M | 2.7 | 97.3 | / | 5.1 | 91.8 | 3.1 | 0.09 | 97.3 | 2.6 |
| 40°C 1W | 23.9 | 75.9 | 0.11 | 2.7 | 93.3 | 3.9 | / | 96.5 | 3.6 |
| 40°C 2W | 32.5 | 67.4 | 0.09 | 4.8 | 90.0 | 5.2 | 0.19 | 95.6 | 4.2 |
| Note: "/" means not detected. | | | | | | | | | |

Table 1.6 Viscosity and osmotic pressure data of formulations A2-A4

| Physical and chemical parameters | A2 | A3 | A4 |
|---|---|---|---|
| Viscosity (cP), 25°C | 7.0 | 5.7 | 29.7 |
| Osmotic pressure (mOsmol/kg) | 353 | 346 | 410 |

Test example A1 and test example A2 were schemes with buffering system, and test example A3 and test example A4 were schemes with self-buffering system. The experimental results showed that under the conditions of oscillating at room temperature (25 ± 2°C) for one week, three freeze-thaw cycles, and storing at 2-8°C for six months, the samples of test example A1, test example A2 and test example A3 were relatively stable, and the purity of SEC-HPLC showed no significant change. Under the condition of storing at 25°C for 1 month, the purity of test example A1, test example A2, test example A3 and test example A4 showed no significant change except the purity of nr-SDS-PAGE.

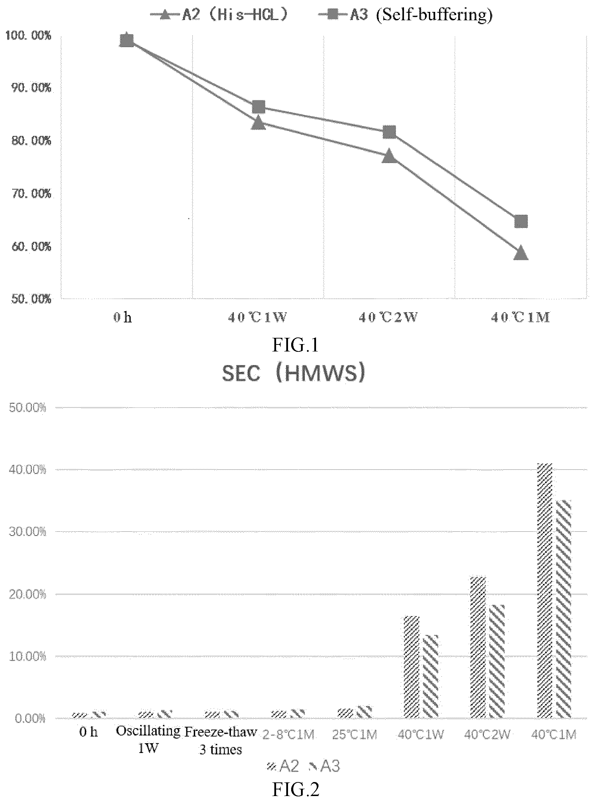

Under the condition of storing at 40 ± 2°C for 1 month, the test results were shown in Tables 1.2-1.4. The trend diagram of SEC main peak purity transformation of formulations A2 and A3 at 40 ± 2°C is shown in FIG. 1, and the trend diagram of SEC polymer purity transformation of formulations A2 and A3 under different storing conditions was shown in FIG. 2. From the above results, the SEC-HPLC purity of test example A3 was greatly improved compared with that of test example A2. Specifically, in test example A3, the purity of the main peak of SEC-HPLC was increased by 5.9% and the aggregate was decreased by 5.9% compared with test example A2. In industry, it was very difficult to improve the stability of protein, the improvement of 1-2% purity was of great significance, and the improvement of 5.9% purity was a very significant breakthrough. In addition, in the preparation process, the high-concentration formulation of the self-buffering system saved the cost of adjuvants. In conclusion, the test results of test examples A1-A4 showed that the self-buffering formulation without histidine and phosphate buffer of the present application showed excellent stability in the whole research process.

Table 1.7 Insoluble particles-MFI results

| Sample Name | MFI | | | | |
|---|---|---|---|---|---|
| | ≥2 μm (particle(s)/mL) | ≥5 μm (particle(s)/mL) | ≥10 μm (particle(s)/mL) | ≥25 μm (particle(s)/mL) | ≥50 μm (particle(s)/mL) |
| A1 | 1798 | 347 | 67 | 5 | 0 |
| A2 | 1216 | 260 | 78 | 5 | 0 |
| A3 | 826 | 117 | 14 | 0 | 0 |

Table 1.8 Insoluble particles-MFI results (Morphological Analysis)

| Sample Name | MFI morphological analysis ≥5 μm (particle(s)/mL) | | | | |
|---|---|---|---|---|---|
| | Silicone oil | Bubble | Dense aggregate | Protein | Packing material |
| A1 | 7 | 10 | 0 | 331 | 0 |
| A2 | 5 | 7 | 0 | 241 | 7 |
| A3 | 5 | 0 | 0 | 113 | 0 |

Tables 1.7 and 1.8 showed the MFI results of insoluble particles. For ocular intravitreal injection products, the reduction of aggregates was expected to reduce a series of adverse reactions such as immunogenicity in patients. Compared with test examples A1 and A2, the purity of nr-SDS-PAGE and rCE-SDS in test example A3 was improved. At the same time, compared with A1 and A2, the insoluble particles in test example A3 were improved. In the analysis of insoluble particles of ≥2 μm, ≥5 μm, ≥10 μm and ≥25 μm, formulation A3 showed lower concentrations of insoluble particles. This was a surprising result, for ocular administration, the improvement of insoluble particles can reduce the adverse reaction rate of patients caused by injection. Thus, the test results showed that the self-buffering formulation of the present application showed excellent prospects in ocular administration.

**Example 2: Stability comparison of self-buffering system formulations with different concentrations of L-arginine hydrochloride**

Test examples B1-B7 were self-buffering formulations with different concentrations of L-arginine hydrochloride, and the concentrations of each component were shown in Table 2.1, among which the concentration of aflibercept in B1-B7 was about 114.3 mg/mL. In example 2, the effects of L-arginine hydrochloride of 20 mM, 40 mM, 50 mM, 60 mM, 70 mM, 80 mM, and 100 mM on the stability of pharmaceutical composition were tested.

Table 2.1 Formulations comprising different concentrations of L-arginine hydrochloride

| Test Examples | Composition | Viscosity (25°C) | Osmotic pressure (mOsmol/kg) |
|---|---|---|---|
| B1 | Aflibercept, 6% w/v sucrose, 0.03% w/v polysorbate20, pH 5.8, 20 mM L-arginine hydrochloride | 8.0 cP | 313 |
| B2 | Aflibercept, 6% w/v sucrose, 0.03% w/v polysorbate20, pH 5.8, 40 mM L-arginine hydrochloride | 8.5 cP | 365 |

(continued)

| Test Examples | Composition | Viscosity (25°C) | Osmotic pressure (mOsmol/kg) |
|---|---|---|---|
| B3 | Aflibercept, 6% w/v sucrose, 0.03% w/v polysorbate20, pH 5.8, 50 mM L-arginine hydrochloride | 8.5 cP | 379 |
| B4 | Aflibercept, 6% w/v sucrose, 0.03% w/v polysorbate20, pH 5.8, 60 mM L-arginine hydrochloride | 8.6 cP | 394 |
| B5 | Aflibercept, 6% w/v sucrose, 0.03% w/v polysorbate20, pH 5.8, 70 mM L-arginine hydrochloride | 8.6 cP | 418 |
| B6 | Aflibercept, 6% w/v sucrose, 0.03% w/v polysorbate20, pH 5.8, 80 mM L-arginine hydrochloride | 8.0 cP | 433 |
| B7 | Aflibercept, 6% w/v sucrose, 0.03% w/v polysorbate20, pH 5.8, 100 mM L-arginine hydrochloride | 7.8 cP | 472 |

Table 2.2 Stability data of formulation B1

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.1 | 98.9 | / | 2.0 | 97.2 | 0.73 | / | 98.0 | 2.0 |
| 37°C 2W | 4.1 | 95.9 | / | 4.6 | 92.4 | 3.0 | / | 97.0 | 3.0 |
| 37°C 1M | 8.4 | 91.4 | 0.25 | / | / | / | / | / | / |
| 25°C 1M | 1.9 | 98.1 | / | / | / | / | / | / | / |
| 2-8°C 3M | 1.5 | 98.5 | / | 2.1 | 96.8 | 1.0 | / | 98.1 | 2.0 |
| Note: "/" means not detected. | | | | | | | | | |

Table 2.3 Stability data of formulation B2

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.4 | 98.6 | / | 2.5 | 96.1 | 1.3 | / | 97.6 | 2.4 |
| 37°C 2W | 5.1 | 94.7 | 0.18 | 3.2 | 93.0 | 3.8 | / | 96.8 | 3.2 |
| 37°C 1M | 9.3 | 90.3 | 0.36 | 3.4 | 91.4 | 5.2 | / | 95.7 | 4.3 |
| 25°C 1M | 1.6 | 98.4 | / | 2.6 | 95.3 | 2.1 | / | 97.5 | 2.5 |
| 2-8°C 3M | 1.3 | 98.7 | / | 3.1 | 95.6 | 1.3 | / | 97.9 | 2.1 |
| Note: "/" means not detected. | | | | | | | | | |

Table 2.4 Stability data of formulation B3

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.4 | 98.6 | / | 2.6 | 96.1 | 1.2 | / | 97.8 | 2.3 |

(continued)

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 37°C 2W | 5.3 | 94.6 | 0.18 | 3.1 | 92.9 | 4.0 | / | 96.9 | 3.1 |
| 37°C 1M | 9.8 | 89.9 | 0.38 | 3.5 | 91.4 | 5.1 | / | 95.7 | 4.3 |
| 25°C 1M | 1.6 | 98.5 | / | 2.4 | 95.8 | 1.8 | / | 97.4 | 2.6 |
| 2-8°C 3M | 1.2 | 98.8 | / | 3.2 | 95.5 | 1.3 | / | 97.8 | 2.2 |
| Note: "/" means not detected. | | | | | | | | | |

Table 2.5 Stability data of formulation B4

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.3 | 98.7 | / | 2.6 | 96.1 | 1.2 | / | 97.8 | 2.2 |
| 37°C 2W | 5.5 | 94.3 | 0.2 | 3.3 | 92.6 | 4.1 | / | 96.9 | 3.1 |
| 37°C 1M | 10.0 | 89.7 | 0.37 | 3.6 | 91.2 | 5.1 | / | 95.8 | 4.3 |
| 25°C 1M | 1.6 | 98.4 | / | 2.6 | 95.3 | 2.0 | / | 97.5 | 2.5 |
| 2-8°C 3M | 1.3 | 98.7 | / | 3.1 | 95.7 | 1.2 | / | 97.9 | 2.1 |
| Note: "/" means not detected. | | | | | | | | | |

Table 2.6 Stability data of formulation B5

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.3 | 98.7 | / | 2.5 | 96.4 | 1.2 | / | 97.8 | 2.2 |
| 37°C 2W | 5.6 | 94.2 | 0.19 | 3.5 | 92.8 | 3.7 | / | 96.9 | 3.1 |
| 37°C 1M | 10.6 | 89.1 | 0.36 | 3.2 | 91.6 | 5.2 | / | 95.6 | 4.4 |
| 25°C 1M | 1.5 | 98.5 | / | 2.9 | 95.1 | 2.0 | / | 97.4 | 2.6 |
| 2-8°C 3M | 1.2 | 98.8 | / | 3.1 | 95.7 | 1.1 | / | 97.9 | 2.1 |
| Note: "/" means not detected. | | | | | | | | | |

Table 2.7 Stability data of formulation B6

| Condition | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.3 | 98.7 | / | 2.4 | 96.4 | 1.2 | / | 97.8 | 2.2 |
| 37°C 2W | 5.9 | 94 | 0.18 | 3.5 | 92.5 | 3.9 | / | 96.9 | 3.1 |
| 37°C 1M | 11.0 | 88.6 | 0.37 | 3.3 | 91.4 | 5.2 | / | 95.6 | 4.4 |
| 25°C 1M | 1.6 | 98.4 | / | 2.8 | 95.1 | 2.0 | / | 97.4 | 2.6 |
| 2-8°C 3M | 1.3 | 98.7 | / | 3.6 | 95.8 | 0.51 | / | 97.8 | 2.2 |
| Note: "/" means not detected. | | | | | | | | | |

Table 2.8 Stability data of formulation B7

| Condition | SEC-HPLC | | | nr-SDS-PAGE | | | rCE-SDS | | |
|---|---|---|---|---|---|---|---|---|---|
| | =~ HMWS (%) | Monomer (%) | LMWS (%) | HMWS (%) | MAIN (%) | LMWS (%) | HMWS (%) | MAIN (%) | LMWS (%) |
| 0 h | 1.0 | 99.0 | / | 2.0 | 97.4 | 0.61 | / | 98.0 | 2.0 |
| 37°C 2W | 5.5 | 94.5 | / | 4.2 | 92.7 | 3.1 | / | 97.0 | 3.0 |
| 37°C 1M | 11.6 | 88.1 | 0.27 | / | / | / | / | / | / |
| 25°C 1M | 1.8 | 98.2 | / | / | / | / | / | / | / |
| 2-8°C 3M | 1.4 | 98.6 | / | 2.2 | 96.8 | 1.0 | / | 98.0 | 2.0 |
| Note: "/" means not detected. | | | | | | | | | |

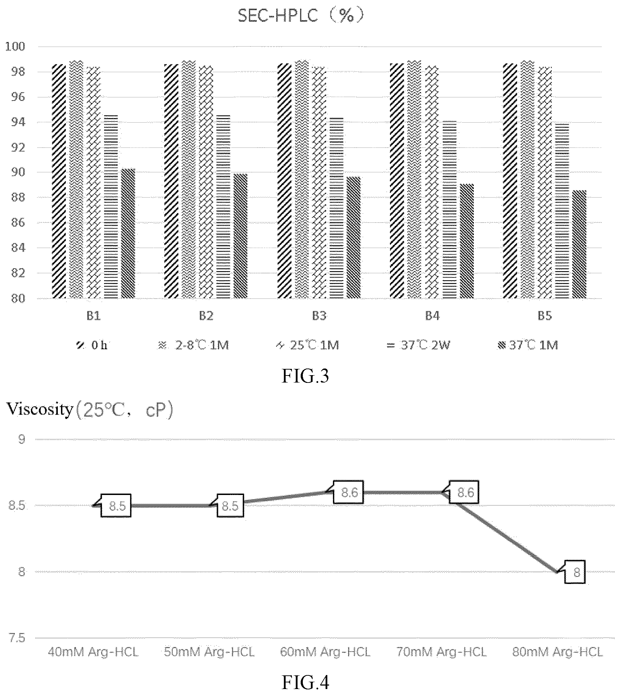

SEC-HPLC, nr-SDS-PAGE and rCE-SDS were used to evaluate the physical stability of L-arginine hydrochloride of self-buffering formulations with different concentrations at 2-8°C, 25 ± 2°C and 37 ± 2°C. The experimental results were shown in Tables 2.2-2.8 and FIGs. 3-4, in which FIG. 3 is a trend diagram of SEC main peak purity transformation of formulations B1, B2, B3, B4 and B5 under different storage conditions, and FIG. 4 is a curve graph of viscosities corresponding to formulations B1, B2, B3, B4 and B5.

The above test results showed that the pharmaceutical compositions comprising L-arginine hydrochloride of 20 mM, 40 mM, 50 mM, 60 mM, 70 mM, 80 mM, and 100 mM showed excellent stability under the conditions of 2-8°C 3M and 25°C 1M, and their purity showed no significant change compared with that at the initial stage (0 h). Meanwhile, L-arginine hydrochloride at different concentrations has no significant effect on the viscosity of the formulations.

Example 3: **Stability comparison of self-buffering system formulations comprising different types and concentrations of stabilizers**

Test examples C1-C10 were self-buffering formulations comprising different types and concentrations of stabilizers, in which the concentration of aflibercept is about 114.3 mg/mL, and the concentrations of each component were shown in Table 3.1. Example 3 examined the effects of different types and concentrations of stabilizers on the stability of pharmaceutical compositions.

Table 3.1 Formulations comprising aflibercept

| Test Example s | Aflibercept (mg/mL) | Polysorbate 20 (% w/v) | L-arginine hydrochloride (mM) | Sucrose (% w/v) | Trehalose (% w/v) | Proline (% w/v) | Lysine hydrochloride (mM) | Sorbitol (% w/v) | Sodium chloride (mM) | pH |
|---|---|---|---|---|---|---|---|---|---|---|
| C1 | 114.3 | 0.03 | 50 | 5 | - | - | - | - | - | 5.8 |
| C2 | 114.3 | 0.03 | 50 | 6 | - | - | - | - | - | 5.8 |
| C3 | 114.3 | 0.03 | 50 | 7 | - | - | - | - | - | 5.8 |
| C4 | 114.3 | 0.03 | 50 | - | 5.5 | - | - | - | - | 5.8 |
| C5 | 114.3 | 0.03 | 50 | - | 6.6 | - | - | - | - | 5.8 |
| C6 | 114.3 | 0.03 | 50 | - | 7.7 | - | - | - | - | 5.8 |
| C7 | 114.3 | 0.03 | 50 | - | - | 2.5 | - | - | - | 5.8 |
| C8 | 114.3 | 0.03 | 50 | - | - | - | 100 | - | - | 5.8 |
| C9 | 114.3 | 0.03 | 50 | - | - | - | - | 4 | - | 5.8 |
| C10 | 114.3 | 0.03 | - | 6.84 | - | - | - | - | 40 | 5.8 |
| Note: "-" means not added. | | | | | | | | | | |

EP 4 588 469 A1

Table 3.2 Stability data of formulation C1

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.3 | 98.7 | / | 3.4 | 94.4 | 2.2 | / | 97.9 | 2.2 |
| 37°C 2W | 6.6 | 93.1 | 0.37 | 3.7 | 92.1 | 4.2 | / | 96.4 | 3.6 |
| 37°C 1M | 13.3 | 86.1 | 0.59 | 5.6 | 87.6 | 6.8 | / | 94.7 | 5.3 |
| 2-8°C 1M | 0.93 | 99.1 | / | 3.6 | 94.5 | 1.9 | / | 97.8 | 2.3 |
| 25°C 1M | 1.4 | 98.2 | 0.42 | 3.8 | 93.6 | 2.5 | / | 97.0 | 3.1 |
| Note: "/" means not detected. | | | | | | | | | |

Table 3.3 Stability data of formulation C2

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.3 | 98.7 | / | 3.0 | 93.2 | 3.8 | / | 97.7 | 2.3 |
| 37°C 2W | 5.6 | 94.0 | 0.36 | 3.7 | 92.2 | 4.1 | / | 96.5 | 3.5 |
| 37°C 1M | 11.3 | 88.1 | 0.56 | 5.1 | 88.0 | 6.9 | / | 94.6 | 5.4 |
| 2-8°C 1M | 0.93 | 99.1 | / | 3.6 | 94.3 | 2.0 | / | 97.7 | 2.3 |
| 25°C 1M | 1.4 | 98.2 | 0.42 | 4.5 | 93.1 | 2.3 | / | 96.9 | 3.1 |
| Note: "/" means not detected. | | | | | | | | | |

Table 3.4 Stability data of formulation C3

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.3 | 98.7 | / | 3.3 | 94.2 | 2.5 | / | 97.8 | 2.2 |
| 37°C 2W | 5.2 | 94.4 | 0.38 | 3.5 | 92.2 | 4.3 | / | 96.5 | 3.5 |
| 37°C 1M | 10.6 | 88.8 | 0.57 | 5.2 | 87.9 | 7.0 | / | 94.3 | 5.7 |
| 2-8°C 1M | 0.95 | 99 | / | 3.7 | 94.3 | 2.0 | / | 97.7 | 2.3 |
| 25°C 1M | 1.3 | 98.2 | 0.41 | 3.5 | 93.9 | 2.5 | / | 96.9 | 3.1 |
| Note: "/" means not detected. | | | | | | | | | |

Table 3.5 Stability data of formulation C4

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.3 | 98.7 | / | 2.8 | 94.1 | 3.0 | / | 98.3 | 1.7 |
| 37°C 2W | 5.5 | 94.2 | 0.32 | 2.4 | 92.9 | 4.7 | / | 96.7 | 3.3 |
| 37°C 1M | 10.8 | 88.7 | 0.46 | 4.0 | 88.0 | 7.9 | / | 94.9 | 5.2 |
| 2-8°C 1M | 0.92 | 99.1 | / | 3.2 | 95.1 | 1.6 | / | 98.1 | 1.9 |

(continued)

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 25°C 1M | 1.4 | 98.3 | 0.35 | 3.1 | 93.9 | 2.9 | / | 97.1 | 2.9 |
| Note: "/" means not detected. | | | | | | | | | |

Table 3.6 Stability data of formulation C5

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.2 | 98.8 | / | 3.2 | 94.9 | 1.8 | / | 98.2 | 1.8 |
| 37°C 2W | 4.8 | 94.9 | 0.28 | 2.3 | 93.0 | 4.7 | / | 96.7 | 3.3 |
| 37°C 1M | 9.7 | 89.9 | 0.44 | 4.6 | 88.0 | 7.4 | / | 94.7 | 5.3 |
| 2-8°C 1M | 0.94 | 99.1 | / | 2.9 | 95.7 | 1.3 | / | 98.1 | 1.9 |
| 25°C 1M | 1.3 | 98.4 | 0.33 | 3.3 | 93.8 | 2.8 | / | 97.0 | 3.1 |
| Note: "/" means not detected. | | | | | | | | | |

Table 3.7 Stability data of formulation C6

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.2 | 98.8 | / | 2.9 | 96.1 | 0.86 | / | 98.1 | 1.9 |
| 37°C 2W | 4.3 | 95.4 | 0.30 | 2.5 | 93.8 | 3.7 | / | 96.6 | 3.4 |
| 37°C 1M | 8.6 | 91 | 0.44 | 3.5 | 89.2 | 7.3 | / | 94.8 | 5.2 |
| 2-8°C 1M | 0.88 | 99.1 | / | 2.9 | 95.8 | 1.3 | / | 98.0 | 2.0 |
| 25°C 1M | 1.3 | 98.4 | 0.34 | 3.2 | 93.7 | 3.0 | / | 97.2 | 2.8 |
| Note: "/" means not detected. | | | | | | | | | |

Table 3.8 Stability data of formulation C7

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.1 | 98.9 | / | 3.1 | 95.3 | 1.5 | / | 98.3 | 1.7 |
| 37°C 2W | 9.9 | 89.8 | 0.30 | 2.1 | 92.6 | 5.3 | / | 95.7 | 4.3 |
| 37°C 1M | 21.1 | 78.4 | 0.47 | 3.3 | 85.4 | 11.3 | / | 92.8 | 7.2 |
| 2-8°C 1M | 0.61 | 99.4 | / | 2.7 | 95.6 | 1.6 | / | 97.9 | 2.1 |
| 25°C 1M | 1.4 | 98.1 | 0.45 | 3.2 | 92.6 | 4.1 | / | 96.5 | 3.5 |
| Note: "/" means not detected. | | | | | | | | | |

Table 3.9 Stability data of formulation C8

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.1 | 98.9 | / | 1.8 | 97.5 | 0.62 | / | 98.1 | 1.9 |
| 37°C 2W | 6.4 | 93.6 | / | 3.1 | 91.8 | 5.1 | / | 96.6 | 3.4 |
| 37°C 1M | 32.4 | 67.3 | 0.35 | 4.0 | 79.4 | 16.6 | / | 89.7 | 10.3 |
| 2-8°C 1M | 1.0 | 99.0 | / | 2.1 | 96.8 | 1.0 | / | 98.0 | 2.0 |
| 25°C 1M | 2.2 | 97.8 | / | 2.2 | 91.6 | 6.1 | / | 95.6 | 4.4 |
| Note: "/" means not detected. | | | | | | | | | |

Table 3.10 Stability data of formulation C9

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.1 | 98.9 | / | 1.5 | 97.7 | 0.71 | / | 98.0 | 2.0 |
| 37°C 2W | 15.4 | 84.6 | / | 2.9 | 89.8 | 7.3 | / | 94.1 | 5.9 |
| 37°C 1M | 14.4 | 85.1 | 0.43 | 4.2 | 88.6 | 7.2 | / | 95.0 | 5.0 |
| 2-8°C 1M | 1.1 | 98.9 | / | 2.3 | 96.9 | 0.81 | / | 98.2 | 1.8 |
| 25°C 1M | 1.6 | 98.4 | / | 2.6 | 95.2 | 2.1 | / | 97.5 | 2.5 |
| Note: "/" means not detected. | | | | | | | | | |

Table 3.11 Stability data of formulation C10

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.1 | 98.9 | / | 1.7 | 97.7 | 0.54 | / | 98.2 | 1.8 |
| 37°C 2W | 5.0 | 95.0 | / | 3.2 | 92.3 | 4.5 | / | 96.1 | 3.9 |
| 37°C 1M | 12.1 | 87.6 | 0.36 | 5.6 | 85.0 | 9.4 | / | 93.8 | 6.2 |
| 2-8°C 1M | 1.1 | 98.9 | / | 2.1 | 96.9 | 0.92 | / | 98.2 | 1.8 |
| 25°C 1M | 1.6 | 98.4 | / | 2.9 | 94.3 | 2.7 | / | 97.0 | 3.0 |
| Note: "/" means not detected. | | | | | | | | | |

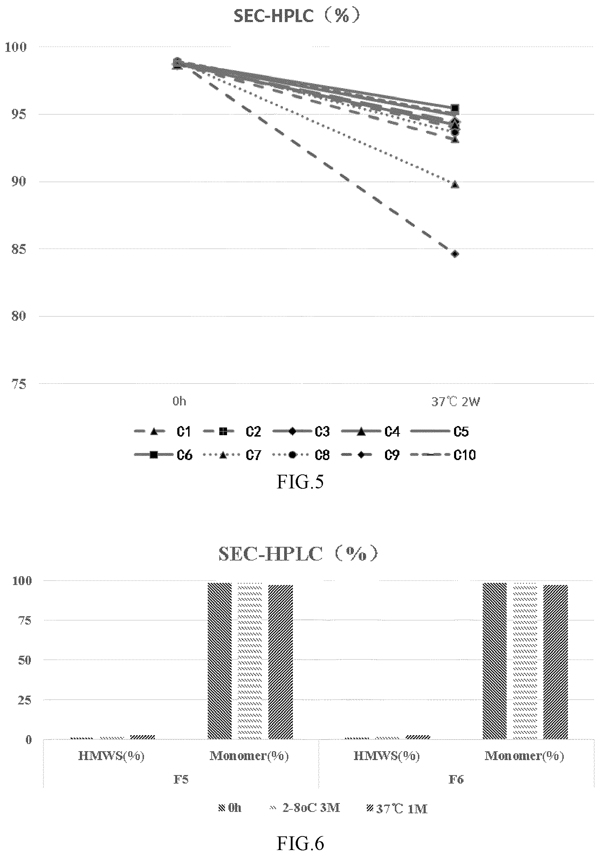

Tables 3.2-3.11 showed the percentage results of Monomer/MAIN (main peak), HMWS (polymer), and fragment (LMWS) of SEC-HPLC, rCE-SDS, and nr-SDS-PAGE of formulations C1-C10 at 0 h, 37 ± 2°C, 2-8°C and 25 ± 2°C. Fig. 5 showed the trend of the SEC main peak purity transformation of formulations C1-C10 at 37 ± 2°C. From the above results, it can be seen that ten of high-purity self-buffering high-concentration formulations of C1-C10 were prepared by adding sucrose, trehalose, sorbitol, proline, lysine hydrochloride and other adjuvants, and the purity of the main peak of 0 h SEC-HPLC was above 98.7%. The results of 37°C 1 M showed that the purity of the main peak of SEC-HPLC of formulations C1-C3 was between 86.1% and 88.8%, the purity of SEC-HPLC main peak of formulations C4-C6 was between 88.7% and 91.0%, and the purity of SEC-HPLC main peak of formulations C7, C8 and C9 were 78.4%, 67.3% and 85.1% respectively. rCE-SDS and nr-SDS-PAGE showed no significant difference in purity of each formulation.

The above experimental results showed that sucrose and trehalose had obvious protective effects on aflibercept. Further exploring the concentration range of sucrose and trehalose, it was found that the pharmaceutical composition had excellent stability when the sucrose concentration is within the range of 5-7% w/v and the trehalose concentration is within the range of 5.5-7.7% w/v.

**Example 4: Stability comparison of different self-buffering system formulations**

The pH value, protein concentration and polysorbate 20 concentration of different self-buffering system formulations were adjusted, and each component was shown in Table 4.1. The stability of the aflibercept formulation was tested under the following conditions: 6% w/v of sucrose, 50 mM of L-arginine hydrochloride, pH value of 5.5, 5.8, and 6.1 respectively, protein concentration of about 100 mg/mL, about 114.3 mg/mL, and about 125 mg/mL respectively, and 0.01% w/v, 0.03% w/v, 0.05% w/v of polysorbate 20 respectively.

Table 4.1 Formulations comprising aflibercept

| Test Examples | pH | Protein concentration (mg/mL) | Polysorbate 20 (% w/v) | Sucrose (% w/v) | L-arginine hydrochloride (mM) |
|---|---|---|---|---|---|
| D1 | 6.1 | 125 | 0.01 | 6 | 50 |
| D2 | 5.5 | 100 | 0.05 | 6 | 50 |
| D3 | 5.8 | 100 | 0.01 | 6 | 50 |
| D4 | 5.8 | 114.3 | 0.03 | 6 | 50 |
| D5 | 5.8 | 114.3 | 0.03 | 6 | 50 |
| D6 | 5.5 | 125 | 0.05 | 6 | 50 |
| D7 | 6.1 | 100 | 0.05 | 6 | 50 |
| D8 | 5.5 | 125 | 0.01 | 6 | 50 |
| D9 | 5.5 | 100 | 0.01 | 6 | 50 |
| D10 | 6.1 | 125 | 0.05 | 6 | 50 |

Table 4.2 Stability data of formulation D1

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.83 | 99.2 | / | 2.9 | 96.5 | 0.54 | / | 98.3 | 1.7 |
| 37°C 1W | 3.3 | 96.7 | / | 2.6 | 95.5 | 1.8 | / | 97.7 | 2.3 |
| 37°C 1M | 13 | 86.6 | 0.36 | 5.1 | 86.7 | 8.1 | / | 95.1 | 4.9 |
| 2-8°C 1M | 1.5 | 98.5 | / | 3.0 | 96.2 | 0.82 | / | 98.3 | 1.7 |
| 25°C 1M | 2.3 | 97.7 | / | 3.2 | 94.5 | 2.3 | / | 97.5 | 2.5 |
| Note: "/" means not detected. | | | | | | | | | |

Table 4.3 Stability data of formulation D2

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.77 | 99.2 | / | 2.4 | 97.1 | 0.45 | / | 98.3 | 1.7 |
| 37°C 1W | 3.5 | 96.5 | / | 2.1 | 96.0 | 1.9 | / | 97.6 | 2.4 |
| 37°C 1M | 14.5 | 85 | 0.48 | 3.9 | 87.4 | 8.7 | / | 95.0 | 5.0 |
| 2-8°C 1M | 1.1 | 98.9 | / | 2.2 | 96.7 | 1.1 | / | 98.3 | 1.7 |
| 25°C 1M | 1.8 | 98.2 | / | 2.1 | 95.5 | 2.3 | / | 97.5 | 2.5 |
| Note: "/" means not detected. | | | | | | | | | |

Table 4.4 Stability data of formulation D3

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.89 | 99.1 | / | 2.9 | 96.6 | 0.51 | / | 98.3 | 1.7 |
| 37°C 1W | 2.8 | 97.2 | / | 2.4 | 95.8 | 1.8 | / | 97.4 | 2.6 |
| 37°C 1M | 10 | 89.6 | 0.38 | 4.9 | 86.5 | 8.6 | / | 95.2 | 4.8 |
| 2-8°C 1M | 1.4 | 98.6 | / | 2.8 | 96.2 | 0.97 | / | 98.3 | 1.7 |
| 25°C 1M | 2 | 98 | / | 2.6 | 95.3 | 2.1 | / | 97.5 | 2.5 |
| Note: "/" means not detected. | | | | | | | | | |

Table 4.5 Stability data of formulation D4

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.84 | 99.2 | / | 2.4 | 96.4 | 1.1 | / | 98.1 | 1.9 |
| 37°C 1W | 3.5 | 96.5 | / | 2.4 | 95.5 | 2.0 | / | 97.7 | 2.3 |
| 37°C 1M | 14.2 | 85.4 | 0.4 | 5.0 | 86.9 | 8.1 | / | 95.2 | 4.8 |
| 2-8°C 1M | 1.3 | 98.7 | / | 2.6 | 96.4 | 0.97 | / | 98.2 | 1.8 |
| 25°C 1M | 2 | 98 | / | 2.4 | 95.6 | 2.0 | / | 97.4 | 2.6 |
| Note: "/" means not detected. | | | | | | | | | |

Table 4.6 Stability data of formulation D5

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.86 | 99.1 | / | 2.2 | 96.9 | 0.86 | / | 98.2 | 1.8 |
| 37°C 1W | 3.5 | 96.5 | / | 2.5 | 95.8 | 1.6 | / | 97.7 | 2.3 |
| 37°C 1M | 13.9 | 85.7 | 0.45 | 4.9 | 87.1 | 8.0 | / | 95.3 | 4.7 |
| 2-8°C 1M | 1.4 | 98.6 | / | 2.6 | 96.6 | 0.80 | / | 98.3 | 1.7 |
| 25°C 1M | 1.9 | 98.1 | / | 2.6 | 95.4 | 2.0 | / | 97.4 | 2.6 |
| Note: "/" means not detected. | | | | | | | | | |

Table 4.7 Stability data of formulation D6

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.83 | 99.2 | / | 2.2 | 96.7 | 1.0 | / | 98.3 | 1.7 |
| 37°C 1W | 4.7 | 95.3 | / | 2.0 | 95.9 | 2.1 | / | 97.5 | 2.6 |
| 37°C 1M | 19.7 | 79.8 | 0.46 | 4.0 | 86.8 | 9.1 | / | 94.9 | 5.1 |
| 2-8°C 1M | 1.3 | 98.7 | / | 2.4 | 96.4 | 1.1 | / | 98.3 | 1.7 |

(continued)

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 25°C 1M | 2.1 | 97.9 | / | 2.7 | 94.9 | 2.3 | / | 97.4 | 2.7 |
| Note: "/" means not detected. | | | | | | | | | |

Table 4.8 Stability data of formulation D7

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.95 | 99.0 | / | 2.6 | 96.4 | 1.0 | / | 98.2 | 1.8 |
| 37°C 1W | 3.0 | 97.0 | / | 2.1 | 95.5 | 2.4 | / | 97.4 | 2.6 |
| 37°C 1M | 10.9 | 88.7 | 0.37 | 5.1 | 86.3 | 8.5 | / | 95.3 | 4.8 |
| 2-8°C 1M | 1.5 | 98.5 | / | 2.9 | 96.2 | 0.89 | / | 98.0 | 2.0 |
| 25°C 1M | 1.9 | 98.1 | / | 3.3 | 94.7 | 1.9 | / | 97.2 | 2.8 |
| Note: "/" means not detected. | | | | | | | | | |

Table 4.9 Stability data of formulation D8

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.87 | 99.1 | / | 2.3 | 96.7 | 0.97 | / | 98.2 | 1.8 |
| 37°C 1W | 4.3 | 95.7 | / | 2.0 | 95.8 | 2.1 | / | 97.4 | 2.6 |
| 37°C 1M | 19.3 | 80.3 | 0.44 | 4.3 | 86.2 | 9.4 | / | 95.2 | 4.8 |
| 2-8°C 1M | 1.4 | 98.6 | / | 2.1 | 96.5 | 1.3 | / | 98.3 | 1.7 |
| 25°C 1M | 2.2 | 97.8 | / | 2.6 | 95.1 | 2.3 | / | 97.3 | 2.8 |
| Note: "/" means not detected. | | | | | | | | | |

Table 4.10 Stability data of formulation D9

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 0.83 | 99.2 | / | 2.0 | 97.2 | 0.67 | / | 98.1 | 1.9 |
| 37°C 1W | 3.3 | 96.7 | / | 1.8 | 96.0 | 2.1 | / | 97.5 | 2.5 |
| 37°C 1M | 14.2 | 85.3 | 0.44 | 4.0 | 86.3 | 9.7 | / | 95.2 | 4.8 |
| 2-8°C 1M | 1.3 | 98.7 | / | 2.3 | 96.5 | 1.1 | / | 98.2 | 1.8 |
| 25°C 1M | 1.7 | 98.3 | / | 2.3 | 95.4 | 2.2 | / | 97.3 | 2.7 |
| Note: "/" means not detected. | | | | | | | | | |

Table 4.11 Stability data of formulation D10

| Conditions | SEC-HPLC | | | nr-SDS-PAGE | | | rCE-SDS | | |
|---|---|---|---|---|---|---|---|---|---|
| | HMWS (%) | Monomer (%) | LMWS (%) | HMWS (%) | MAIN (%) | LMWS (%) | HMWS (%) | MAIN (%) | LMWS (%) |
| 0 h | 1.0 | 99.0 | / | 2.7 | 96.7 | 0.63 | / | 98.2 | 1.8 |
| 37°C 1W | 3.3 | 96.7 | / | 2.6 | 95.5 | 1.9 | / | 97.5 | 2.5 |
| 37°C 1M | 12.7 | 87 | 0.32 | 5.5 | 86.0 | 8.4 | / | 95.2 | 4.8 |
| 2-8°C 1M | 1.8 | 98.2 | / | 3.1 | 96.1 | 0.72 | / | 98.2 | 1.8 |
| 25°C 1M | 2.2 | 97.8 | / | 3.2 | 94.7 | 2.0 | / | 97.4 | 2.7 |
| Note: "/" means not detected. | | | | | | | | | |

Table 4.12 Viscosity and osmotic pressure data of formulations D1-D10

| Formulations | D1 | D2 | D3 | D4 | D5 | D6 | D7 | D8 | D9 | D10 |
|---|---|---|---|---|---|---|---|---|---|---|
| Viscosity (cP, 25°C) | 10.0 | 5.8 | 6.5 | 8.1 | 8.1 | 9.3 | 6.0 | 10.1 | 5.6 | 8.6 |
| Osmotic pressure (mOsmol/kg) | 387 | 342 | 376 | 361 | 363 | 349 | 376 | 348 | 348 | 377 |

The test results were shown in Tables 4.2-4.12. From the above results, it can be seen that the purity of the main peaks of SEC-HPLC of formulations D1-D10 at 0 h was above 99.0%; the results of 2-8°C 1 M and 25°C 1 M showed that the purity of the main peak of SEC-HPLC of formulations D1-D10 was between 97.7% and 98.9%. The viscosities of the formulations were 5.6-10.1 cP, and the osmotic pressure results were 342-387 mOsmol/kg. The above results showed that the stability of high-concentration self-buffering formulation was kept at a good level within the range of protein concentration of 100-125 mg/mL, pH of 5.5-6.1 and polysorbate 20 concentration of 0.01-0.05% w/v.

**Example 5: Formulation dilution Test**

In order to simulate the stability of diluted aflibercept in vitreous body, the stability of formulations with 2 mg/mL and 0.1 mg/mL of aflibercept was tested. Table 5.1 showed the composition information of the two formulations. In order to prepare the formulation solution, the expected adjuvant solution was used for dilution, and the adjuvant to be added was added to the antibody solution in the form of mother liquor. All the formulations were aseptically filtered through a 0.22 μm low protein binding filter, filled into a 2 mL sterile vial under aseptic conditions, and sealed with a film coated rubber stopper and a cap made of aluminum-plastics combinations. SEC-HPLC was used to determine the presence (%) of high molecular weight species (HMWS, LMWS) and main peak (Monomer/MAIN), and to evaluate the stability of aflibercept of the two formulations during storage at 37°C, so as to characterize the stability of the drugs in the vitreous body.

Table 5.1 Formulations comprising aflibercept

| Formulations | pH | Protein concentration (mg/mL) | Glucose injection (mg/mL) | Diluent | Osmotic pressure (mOsmol/kg) |
|---|---|---|---|---|---|
| E1 | 7.39 | 0.1 | 0.4 | 1×PBS solution | 273 |
| E2 | 7.41 | 2 | 0.4 | 1×PBS solution | 274 |

Table 5.2 Stability data of formulation E1

| Conditions | SEC-HPLC | | |
|---|---|---|---|
| | HMWS (%) | Monomer (%) | LMWS (%) |
| 0 h | 0.69 | 98.7 | 0.58 |
| 37°C 1M | 0.55 | 98.8 | 0.62 |
| 37°C 2M | 0.67 | 98.8 | 0.55 |

(continued)

| Conditions | SEC-HPLC | | |
|---|---|---|---|
| | HMWS (%) | Monomer (%) | LMWS (%) |
| 37°C 3M | 0.69 | 98.3 | 1.0 |
| 37°C 4M | 1.2 | 96.5 | 2.3 |

Table 5.3 Stability data of formulation E2

| Condition | SEC-HPLC | | |
|---|---|---|---|
| | HMWS (%) | Monomer (%) | LMWS (%) |
| 0 h | 0.97 | 99 | / |
| 37°C 1M | 1.1 | 98.6 | 0.27 |
| 37°C 2M | 2.1 | 97.5 | 0.42 |
| 37°C 3M | 3.2 | 96.2 | 0.68 |
| 37°C 4M | 4.9 | 93.7 | 1.4 |
| Note: "/" means not detected. | | | |

The test results were shown in Tables 5.2-5.3. From the above results, it can be found that there was no significant change in the purity of the main peak of SEC-HPLC of formulation 1 and formulation 2 after the stability investigation at 37°C 3M, indicating that the simulated diluted aflibercept has a good stability in the vitreous body.

**Example 6: Lyophilized and pH test of formulation**

In order to further confirm the range of pH value and investigate the stability of lyophilized formulation, the stability of the formulation was investigated under the conditions of pH 5.8, 6.0, 6.2, and 6.4 respectively, and samples of lyophilized formulations were prepared at the same time. All formulations were aseptically filtered through a 0.22 $\mu$m low protein binding filter, and filled into a 2 mL sterile vial under aseptic conditions, and sealed with a film coated rubber stopper and a cap made of aluminum-plastics combinations. These formulation solutions were stored in the following conditions: 37°C 1 M and 2-8°C 3 M to investigate the stability of the samples. Table 6.1 showed the information of different formulations, in which the concentration of aflibercept is about 114.3 mg/mL, and Tables 6.2-6.7 showed the stability results of different formulations.

Table 6.1 Formulations comprising aflibercept

| Test Examples | Formulations | Aflibercept (mg/mL) | Polysorbate 20 (% w/v) | L-arginine hydrochloride (mM) | Sucrose (% w/v) | Trehalose (% w/v) | pH |
|---|---|---|---|---|---|---|---|
| F1 | Injection | 114.3 | 0.03 | - | - | 9 | 5.8 |
| F2 | Injection | 114.3 | 0.03 | - | - | 9 | 6.0 |
| F3 | Injection | 114.3 | 0.03 | - | - | 9 | 6.2 |
| F4 | Injection | 114.3 | 0.03 | - | - | 9 | 6.4 |
| F5 | Lyophilized powder | 114.3 | 0.03 | 50 | 6 | - | 5.8 |
| F6 | Lyophilized powder | 114.3 | 0.03 | 50 | - | 6.6 | 5.8 |
| Note: -means not added. | | | | | | | |

Table 6.2 Stability data of formulation F1

| Conditions | SEC-HPLC | | |
|---|---|---|---|
| | HMWS (%) | Monomer (%) | LMWS (%) |
| 0 h | 1.4 | 98.6 | / |
| 2-8°C 3M | 1.7 | 98.4 | / |
| 37°C 1M | 5.4 | 94.6 | / |
| Note: "/" means not detected. | | | |

Table 6.3 Stability data of formulation F2

| Conditions | SEC-HPLC | | |
|---|---|---|---|
| | HMWS (%) | Monomer (%) | LMWS (%) |
| 0 h | 1.5 | 98.5 | / |
| 2-8°C 3M | 1.8 | 98.2 | / |
| 37°C 1M | 5.6 | 94.4 | / |
| Note: "/" means not detected. | | | |

Table 6.4 Stability data of formulation F3

| Conditions | SEC-HPLC | | |
|---|---|---|---|
| | HMWS (%) | Monomer (%) | LMWS (%) |
| 0 h | 1.6 | 98.4 | / |
| 2-8°C 3M | 1.9 | 98.1 | / |
| 37°C 1M | 6.5 | 93.5 | / |
| Note: "/" means not detected. | | | |

Table 6.5 Stability data of formulation F4

| Conditions | SEC-HPLC | | |
|---|---|---|---|
| | HMWS (%) | Monomer (%) | LMWS (%) |
| 0 h | 1.7 | 98.3 | / |
| 2-8°C 3M | 2.0 | 98.0 | / |
| 37°C 1M | 6.6 | 93.4 | / |
| Note: "/" means not detected. | | | |

Table 6.6 Stability data of lyophilized powder of formulation F5

| Conditions | SEC-HPLC | | |
|---|---|---|---|
| | HMWS (%) | Monomer (%) | LMWS (%) |
| 0 h | 1.2 | 98.8 | / |
| 2-8°C 3M | 1.5 | 98.5 | / |
| 37°C 1M | 2.6 | 97.4 | / |
| Note: "/" means not detected. | | | |

Table 6.7 Stability data of lyophilized powder of formulation F6

| Conditions | SEC-HPLC | | |
|---|---|---|---|
| | HMWS (%) | Monomer (%) | LMWS (%) |
| 0 h | 1.2 | 98.8 | / |
| 2-8°C 3M | 1.6 | 98.4 | / |
| 37°C 1M | 2.9 | 97.1 | / |
| Note: "/" means not detected. | | | |

The test results were shown in Tables 6.2-6.7 and FIG. 6, wherein FIG. 6 was the trend diagram of the SEC purity transformation of formulations F5 and F6 at 2-8°C and 37 ± 2°C. The results showed there was no significant difference in the purity of the main peak of SEC-HPLC of formulations F1-F4 after the stability investigation at 2-8°C 3 M and 37°C 1M, indicating that the formulations are stable in the pH ranging from 5.8 to 6.4; at 37°C 1 M, there was no significant difference in the purity of the main peak of SEC-HPLC of formulations F5 and F6, indicating that the lyophilized formulations of the present application have a good stability and feasibility.

The embodiments of the present application described above are only exemplary, and those skilled in the art can recognize or determine the equivalents of innumerable specific compounds, materials and operations without the need for extraordinary experiments. All such equivalents are within the scope of the present application and are encompassed by the claims.

**Claims**

1.   An aqueous pharmaceutical composition, comprising:

1) a VEGF binding molecule, wherein the VEGF binding molecule is selected from the group consisting of a VEGF receptor fusion protein and an anti-VEGF antibody or an antigen-binding fragment thereof, and a concentration of the VEGF binding molecule is at least 100 mg/mL;
2) a stabilizer, wherein the stabilizer is selected from the group consisting of sucrose, trehalose, proline, a pharmaceutically acceptable salt of proline, lysine, a pharmaceutically acceptable salt of lysine, sorbitol, and a combination thereof;
3) a surfactant, wherein the surfactant is selected from the group consisting of polysorbate 80, polysorbate 20, poloxamer, and a combination thereof; and

wherein the aqueous pharmaceutical composition does not comprise a buffering agent and has a pH of 5.0-7.0.

2.   The aqueous pharmaceutical composition according to claim 1, wherein the aqueous pharmaceutical composition further comprises:

4) at least one of L-arginine, a pharmaceutically acceptable salt of L-arginine, or sodium chloride;
preferably, a concentration of L-arginine or the pharmaceutically acceptable salt of L-arginine is 0-100 mM, or a concentration of sodium chloride is 0-40 mM.

3.   The aqueous pharmaceutical composition according to claim 2, wherein the concentration of L-arginine or the pharmaceutically acceptable salt of L-arginine is 20-100 mM, or the concentration of sodium chloride is 40 mM; preferably, the concentration of L-arginine or the pharmaceutically acceptable salt of L-arginine is 20 mM, 40 mM, 50 mM, 60 mM, 70 mM, 80 mM, or 100 mM.

4.   The aqueous pharmaceutical composition according to any one of claims 1-3, wherein the concentration of the VEGF binding molecule in the aqueous pharmaceutical composition is at least 100 mg/mL, at least 114.3 mg/mL, at least 125 mg/mL, or at least 180 mg/mL; preferably, the concentration of the VEGF binding molecule in the aqueous pharmaceutical composition is 100-180 mg/mL, preferably 100-125 mg/mL.

5.   The aqueous pharmaceutical composition according to any one of claims 1-4, wherein a concentration of the stabilizer is 5-10% w/v; preferably, the stabilizer is selected from the group consisting of sucrose and trehalose, a concentration of the sucrose is 5-9% w/v, or a concentration of the trehalose is 5.5-10% w/v.

6. The aqueous pharmaceutical composition according to claim 5, wherein the concentration of sucrose is 5% w/v, 6% w/v, 7% w/v, 8% w/v, or 9% w/v; or
the concentration of trehalose is 5.5% w/v, 6.6% w/v, 7.7% w/v, 8.0% w/v, or 10% w/v.

7. The aqueous pharmaceutical composition according to any one of claims 1-6, wherein a concentration of the surfactant is 0.01-0.2% w/v.

8. The aqueous pharmaceutical composition according to claim 7, wherein the surfactant is polysorbate 20, and a concentration of polysorbate 20 is 0.01% w/v, 0.03% w/v, 0.05% w/v, 0.07% w/v, 0.1% w/v, or 0.2% w/v.

9. The aqueous pharmaceutical composition according to any one of claims 1-8, wherein the pH of the aqueous pharmaceutical composition is 5.5-6.4; preferably, the pH is 5.5, 5.8, 6.0, 6.1, 6.2, or 6.4.

10. The aqueous pharmaceutical composition according to any one of claims 1-9, comprising a concentration of 100-180 mg/mL of the VEGF receptor fusion protein; 5-9% w/v of sucrose or 5.5-10% w/v of trehalose; 0.01-0.2% w/v of polysorbate 20; and 0-100 mM of L-arginine hydrochloride or 0-40 mM of sodium chloride; and wherein the pH of the aqueous pharmaceutical composition is 5.5-7.0.

11. The aqueous pharmaceutical composition according to any one of claims 1-9, comprising a concentration of 100-180 mg/mL of the VEGF receptor fusion protein; 5-7% w/v of sucrose or 5.5-7.7% w/v of trehalose; 0.01-0.2% w/v of polysorbate 20; and 40-80 mM of L-arginine hydrochloride or 0-40 mM of sodium chloride; and wherein the pH of the aqueous pharmaceutical composition is 5.5-6.4.

12. An aqueous pharmaceutical composition selected from the group consisting of:

an aqueous pharmaceutical composition A comprising: a concentration of 114.3 mg/mL of a VEGF receptor fusion protein, 5% w/v of sucrose, 0.03% w/v of polysorbate 20, and 50 mM of L-arginine hydrochloride, wherein a pH of the aqueous pharmaceutical composition is 5.5-6.4;
an aqueous pharmaceutical composition B comprising: a concentration of 114.3 mg/mL of a VEGF receptor fusion protein, 6% w/v of sucrose, 0.03% w/v of polysorbate 20, and 50 mM of L-arginine hydrochloride, wherein a pH of the aqueous pharmaceutical composition is 5.5-6.4;
an aqueous pharmaceutical composition C comprising: a concentration of 114.3 mg/mL of a VEGF receptor fusion protein, 7% w/v of sucrose, 0.03% w/v of polysorbate 20, and 50 mM of L-arginine hydrochloride, wherein a pH of the aqueous pharmaceutical composition is 5.5-6.4;
an aqueous pharmaceutical composition D comprising: a concentration of 114.3 mg/mL of a VEGF receptor fusion protein, 5.5% w/v of trehalose, 0.03% w/v of polysorbate 20, and 50 mM of L-arginine hydrochloride, wherein a pH of the aqueous pharmaceutical composition is 5.5-6.4;
an aqueous pharmaceutical composition E comprising: a concentration of 114.3 mg/mL of a VEGF receptor fusion protein, 6.6% w/v of trehalose, 0.03% w/v of polysorbate 20, and 50 mM of L-arginine hydrochloride, wherein a pH of the aqueous pharmaceutical composition is 5.5-6.4;
an aqueous pharmaceutical composition F comprising: a concentration of 114.3 mg/mL of a VEGF receptor fusion protein, 7.7% w/v of trehalose, 0.03% w/v of polysorbate 20, and 50 mM of L-arginine hydrochloride, wherein a pH of the aqueous pharmaceutical composition is 5.5-6.4;
an aqueous pharmaceutical composition G comprising: a concentration of 114.3 mg/mL of a VEGF receptor fusion protein, 5% w/v of sucrose, 0.03% w/v of polysorbate 20, and 60 mM of L-arginine hydrochloride, wherein a pH of the aqueous pharmaceutical composition is 5.5-6.4;
an aqueous pharmaceutical composition H comprising: a concentration of 114.3 mg/mL of a VEGF receptor fusion protein, 6% w/v of sucrose, 0.03% w/v of polysorbate 20, and 60 mM of L-arginine hydrochloride, wherein a pH of the aqueous pharmaceutical composition is 5.5-6.4;
an aqueous pharmaceutical composition I comprising: a concentration of 114.3 mg/mL of a VEGF receptor fusion protein, 7% w/v of sucrose, 0.03% w/v of polysorbate 20, and 60 mM of L-arginine hydrochloride, wherein a pH of the aqueous pharmaceutical composition is 5.5-6.4;
an aqueous pharmaceutical composition J comprising: a concentration of 114.3 mg/mL of a VEGF receptor fusion protein, 5.5% w/v of trehalose, 0.03% w/v of polysorbate 20, and 60 mM of L-arginine hydrochloride, wherein a pH of the aqueous pharmaceutical composition is 5.5-6.4;
an aqueous pharmaceutical composition K comprising: a concentration of 114.3 mg/mL of a VEGF receptor fusion protein, 6.6% w/v of trehalose, 0.03% w/v of polysorbate 20, and 60 mM of L-arginine hydrochloride, wherein a pH of the aqueous pharmaceutical composition is 5.5-6.4;

an aqueous pharmaceutical composition L comprising: a concentration of 114.3 mg/mL of a VEGF receptor fusion protein, 7.7% w/v of trehalose, 0.03% w/v of polysorbate 20, and 60 mM of L-arginine hydrochloride, wherein a pH of the aqueous pharmaceutical composition is 5.5-6.4;

an aqueous pharmaceutical composition M comprising: a concentration of 114.3 mg/mL of a VEGF receptor fusion protein, 5% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition N comprising: a concentration of 114.3 mg/mL of a VEGF receptor fusion protein, 6% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition O comprising: a concentration of 114.3 mg/mL of a VEGF receptor fusion protein, 7% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition P comprising: a concentration of 114.3 mg/mL of a VEGF receptor fusion protein, 5.5% w/v of trehalose, 0.03% w/v of polysorbate 20, and 70 mM of L-arginine hydrochloride, wherein a pH of the aqueous pharmaceutical composition is 5.5-6.4;

an aqueous pharmaceutical composition Q comprising: a concentration of 114.3 mg/mL of a VEGF receptor fusion protein, 6.6% w/v of trehalose, 0.03% w/v of polysorbate 20, and 70 mM of L-arginine hydrochloride, wherein a pH of the aqueous pharmaceutical composition is 5.5-6.4;

an aqueous pharmaceutical composition R comprising: a concentration of 114.3 mg/mL of a VEGF receptor fusion protein, 7.7% w/v of trehalose, 0.03% w/v of polysorbate 20, and 70 mM of L-arginine hydrochloride, wherein the pH of the aqueous pharmaceutical composition is 5.5-6.4;

an aqueous pharmaceutical composition S comprising: a concentration of 114.3 mg/mL of a VEGF receptor fusion protein, 5% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition T comprising: a concentration of 114.3 mg/mL of a VEGF receptor fusion protein, 6% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition U comprising: a concentration of 114.3 mg/mL of a VEGF receptor fusion protein, 7% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition V comprising: a concentration of 114.3 mg/mL of a VEGF receptor fusion protein, 5.5% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition W comprising: a concentration of 114.3 mg/mL of a VEGF receptor fusion protein, 6.6% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition X comprising: a concentration of 114.3 mg/mL of a VEGF receptor fusion protein, 7.7% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition Y comprising: a concentration of 114.3 mg/mL of a VEGF receptor fusion protein, 5% w/v of sucrose, 0.01% w/v of polysorbate 20, and 50 mM of L-arginine hydrochloride, wherein a pH of the aqueous pharmaceutical composition is 5.5-6.4;

an aqueous pharmaceutical composition Z comprising: a concentration of 114.3 mg/mL of a VEGF receptor fusion protein, 5% w/v of sucrose, 0.05% w/v of polysorbate 20, and 50 mM of L-arginine hydrochloride, wherein a pH of the aqueous pharmaceutical composition is 5.5-6.4;

an aqueous pharmaceutical composition ZA comprising: a concentration of 125 mg/mL of a VEGF receptor fusion protein, 5% w/v of sucrose, 0.03% w/v of polysorbate 20, and 50 mM of L-arginine hydrochloride, wherein a pH of the aqueous pharmaceutical composition is 5.5-6.4;

an aqueous pharmaceutical composition ZB comprising: a concentration of 125 mg/mL of a VEGF receptor fusion protein, 6% w/v of sucrose, 0.03% w/v of polysorbate 20, and 50 mM of L-arginine hydrochloride, wherein a pH of the aqueous pharmaceutical composition is 5.5-6.4;

an aqueous pharmaceutical composition ZC comprises: a concentration of 125 mg/mL of a VEGF receptor fusion protein, 7% w/v of sucrose, 0.03% w/v of polysorbate 20, and 50 mM of L-arginine hydrochloride, wherein a pH of the aqueous pharmaceutical composition is 5.5-6.4;

an aqueous pharmaceutical composition ZD comprising: a concentration of 125 mg/mL of a VEGF receptor fusion protein, 5.5% w/v of trehalose, 0.03% w/v of polysorbate 20, and 50 mM of L-arginine hydrochloride, wherein a pH of the aqueous pharmaceutical composition is 5.5-6.4;

an aqueous pharmaceutical composition ZE comprising: a concentration of 125 mg/mL of a VEGF receptor fusion protein, 6.6% w/v of trehalose, 0.03% w/v of polysorbate 20, and 50 mM of L-arginine hydrochloride, wherein a pH of the aqueous pharmaceutical composition is 5.5-6.4;

an aqueous pharmaceutical composition ZF comprising: a concentration of 125 mg/mL of a VEGF receptor fusion protein, 7.7% w/v of trehalose, 0.03% w/v of polysorbate 20, and 50 mM of L-arginine hydrochloride, wherein a pH of the aqueous pharmaceutical composition is 5.5-6.4;

an aqueous pharmaceutical composition ZG comprising: a concentration of 125 mg/mL of a VEGF receptor fusion protein, 5% w/v of sucrose, 0.03% w/v of polysorbate 20, and 60 mM of L-arginine hydrochloride, wherein a pH of the aqueous pharmaceutical composition is 5.5-6.4;

an aqueous pharmaceutical composition ZH comprising: a concentration of 125 mg/mL of a VEGF receptor fusion protein, 6% w/v of sucrose, 0.03% w/v of polysorbate 20, and 60 mM of L-arginine hydrochloride, wherein a pH of the aqueous pharmaceutical composition is 5.5-6.4;

an aqueous pharmaceutical composition ZI comprising: a concentration of 125 mg/mL of a VEGF receptor fusion protein, 7% w/v of sucrose, 0.03% w/v of polysorbate 20, and 60 mM of L-arginine hydrochloride, wherein a pH of the aqueous pharmaceutical composition is 5.5-6.4;

an aqueous pharmaceutical composition ZJ comprising: a concentration of 125 mg/mL of a VEGF receptor fusion protein, 5.5% w/v of trehalose, 0.03% w/v of polysorbate 20, and 60 mM of L-arginine hydrochloride, wherein a pH of the aqueous pharmaceutical composition is 5.5-6.4;

an aqueous pharmaceutical composition ZK comprising: a concentration of 125 mg/mL of a VEGF receptor fusion protein, 6.6% w/v of trehalose, 0.03% w/v of polysorbate 20, and 60 mM of L-arginine hydrochloride, wherein a pH of the aqueous pharmaceutical composition is 5.5-6.4;

an aqueous pharmaceutical composition ZL comprising: a concentration of 125 mg/mL of a VEGF receptor fusion protein, 7.7% w/v of trehalose, 0.03% w/v of polysorbate 20, and 60 mM of L-arginine hydrochloride, wherein a pH of the aqueous pharmaceutical composition is 5.5-6.4;

an aqueous pharmaceutical composition ZM comprising: a concentration of 125 mg/mL of a VEGF receptor fusion protein, 5% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition ZN comprising: a concentration of 125 mg/mL of a VEGF receptor fusion protein, 6% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition ZO comprising: a concentration of 125 mg/mL of a VEGF receptor fusion protein, 7% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition ZP comprising: a concentration of 125 mg/mL of a VEGF receptor fusion protein, 5.5% w/v of trehalose, 0.03% w/v of polysorbate 20, and 70 mM of L-arginine hydrochloride, wherein a pH of the aqueous pharmaceutical composition is 5.5-6.4;

an aqueous pharmaceutical composition ZQ comprising: a concentration of 125 mg/mL of a VEGF receptor fusion protein, 6.6% w/v of trehalose, 0.03% w/v of polysorbate 20, and 70 mM of L-arginine hydrochloride, wherein a pH of the aqueous pharmaceutical composition is 5.5-6.4;

an aqueous pharmaceutical composition ZR comprising: a concentration of 125 mg/mL of a VEGF receptor fusion protein, 7.7% w/v of trehalose, 0.03% w/v of polysorbate 20, and 70 mM of L-arginine hydrochloride, wherein a pH of the aqueous pharmaceutical composition is 5.5-6.4;

an aqueous pharmaceutical composition ZS comprising: a concentration of 125 mg/mL of a VEGF receptor fusion protein, 5% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition ZT comprising: a concentration of 125 mg/mL of a VEGF receptor fusion protein, 6% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition ZU comprising: a concentration of 125 mg/mL of a VEGF receptor fusion protein, 7% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition ZV comprising: a concentration of 125 mg/mL of a VEGF receptor fusion protein, 5.5% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition ZW comprising: a concentration of 125 mg/mL of a VEGF receptor fusion protein, 6.6% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition ZX comprising: a concentration of 125 mg/mL of a VEGF receptor fusion protein, 7.7% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition ZY comprising: a concentration of 125 mg/mL of a VEGF receptor fusion protein, 5% w/v of sucrose, 0.01% w/v of polysorbate 20, and 50 mM of L-arginine hydrochloride, wherein a pH of the aqueous pharmaceutical composition is 5.5-6.4;

an aqueous pharmaceutical composition ZZ comprising: a concentration of 125 mg/mL of a VEGF receptor fusion protein, 5% w/v of sucrose, 0.05% w/v of polysorbate 20, and 50 mM of L-arginine hydrochloride, wherein a pH of the aqueous pharmaceutical composition is 5.5-6.4;

an aqueous pharmaceutical composition AA comprising: a concentration of 180 mg/mL of a VEGF receptor fusion protein, 5% w/v of sucrose, 0.03% w/v of polysorbate 20, and 50 mM of L-arginine hydrochloride, wherein a pH of the aqueous pharmaceutical composition is 5.5-6.4;

an aqueous pharmaceutical composition AB comprising: a concentration of 180 mg/mL of a VEGF receptor fusion protein, 6% w/v of sucrose, 0.03% w/v of polysorbate 20, and 50 mM of L-arginine hydrochloride, wherein a pH of the aqueous pharmaceutical composition is 5.5-6.4;

an aqueous pharmaceutical composition AC comprising: a concentration of 180 mg/mL of a VEGF receptor fusion protein, 7% w/v of sucrose, 0.03% w/v of polysorbate 20, and 50 mM of L-arginine hydrochloride, wherein a pH of the aqueous pharmaceutical composition is 5.5-6.4;

an aqueous pharmaceutical composition AD comprising: a concentration of 180 mg/mL of a VEGF receptor fusion protein, 5.5% w/v of trehalose, 0.03% w/v of polysorbate 20, and 50 mM of L-arginine hydrochloride, wherein a pH of the aqueous pharmaceutical composition is 5.5-6.4;

an aqueous pharmaceutical composition AE comprising: a concentration of 180 mg/mL of a VEGF receptor fusion protein, 6.6% w/v of trehalose, 0.03% w/v of polysorbate 20, and 50 mM of L-arginine hydrochloride, wherein a pH of the aqueous pharmaceutical composition is 5.5-6.4;

an aqueous pharmaceutical composition AF comprising: a concentration of 180 mg/mL of a VEGF receptor fusion protein, 7.7% w/v of trehalose, 0.03% w/v of polysorbate 20, and 50 mM of L-arginine hydrochloride, wherein a pH of the aqueous pharmaceutical composition is 5.5-6.4;

an aqueous pharmaceutical composition AG comprising: a concentration of 180 mg/mL of a VEGF receptor fusion protein, 5% w/v of sucrose, 0.03% w/v of polysorbate 20, and 60 mM of L-arginine hydrochloride, wherein a pH of the aqueous pharmaceutical composition is 5.5-6.4;

an aqueous pharmaceutical composition AH comprising: a concentration of 180 mg/mL of a VEGF receptor fusion protein, 6% w/v of sucrose, 0.03% w/v of polysorbate 20, and 60 mM of L-arginine hydrochloride, wherein a pH of the aqueous pharmaceutical composition is 5.5-6.4;

an aqueous pharmaceutical composition AI comprising: a concentration of 180 mg/mL of a VEGF receptor fusion protein, 7% w/v of sucrose, 0.03% w/v of polysorbate 20, and 60 mM of L-arginine hydrochloride, wherein a pH of the aqueous pharmaceutical composition is 5.5-6.4;

an aqueous pharmaceutical composition AJ comprising: a concentration of 180 mg/mL of a VEGF receptor fusion protein, 5.5% w/v of trehalose, 0.03% w/v of polysorbate 20, and 60 mM of L-arginine hydrochloride, wherein a pH of the aqueous pharmaceutical composition is 5.5-6.4;

an aqueous pharmaceutical composition AK comprising: a concentration of 180 mg/mL of a VEGF receptor fusion protein, 6.6% w/v of trehalose, 0.03% w/v of polysorbate 20, and 60 mM of L-arginine hydrochloride, wherein a pH of the aqueous pharmaceutical composition is 5.5-6.4;

an aqueous pharmaceutical composition AL comprising: a concentration of 180 mg/mL of a VEGF receptor fusion protein, 7.7% w/v of trehalose, 0.03% w/v of polysorbate 20, and 60 mM of L-arginine hydrochloride, wherein a pH of the aqueous pharmaceutical composition is 5.5-6.4;

an aqueous pharmaceutical composition AM comprising: a concentration of 180 mg/mL of a VEGF receptor fusion protein, 5% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition AN comprising: a concentration of 180 mg/mL of a VEGF receptor fusion protein, 6% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition AO comprising: a concentration of 180 mg/mL of a VEGF receptor fusion protein, 7% w/v of sucrose, 0.03% w/v of polysorbate 20, and 70 mM of L-arginine hydrochloride, wherein a pH of the aqueous pharmaceutical composition is 5.5-6.4;

an aqueous pharmaceutical composition AP comprising: a concentration of 180 mg/mL of a VEGF receptor fusion protein, 5.5% w/v of trehalose, 0.03% w/v of polysorbate 20, and 70 mM of L-arginine hydrochloride, wherein a pH of the aqueous pharmaceutical composition is 5.5-6.4;

an aqueous pharmaceutical composition AQ comprising: a concentration of 180 mg/mL of a VEGF receptor fusion protein, 6.6% w/v of trehalose, 0.03% w/v of polysorbate 20, and 70 mM of L-arginine hydrochloride, wherein a pH of the aqueous pharmaceutical composition is 5.5-6.4;

an aqueous pharmaceutical composition AR comprising: a concentration of 180 mg/mL of a VEGF receptor fusion protein, 7.7% w/v of trehalose, 0.03% w/v of polysorbate 20, and 70 mM of L-arginine hydrochloride, wherein a pH of the aqueous pharmaceutical composition is 5.5-6.4;

an aqueous pharmaceutical composition AS comprising: a concentration of 180 mg/mL of a VEGF receptor fusion protein, 5% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition AT comprising: a concentration of 180 mg/mL of a VEGF receptor fusion protein, 6% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition AU comprising: a concentration of 180 mg/mL of a VEGF receptor fusion protein, 7% w/v of sucrose, 0.03% w/v of polysorbate 20, and 80 mM of L-arginine hydrochloride, wherein a pH of the aqueous pharmaceutical composition is 5.5-6.4;

an aqueous pharmaceutical composition AV comprising: a concentration of 180 mg/mL of a VEGF receptor fusion protein, 5.5% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition AW comprising: a concentration of 180 mg/mL of a VEGF receptor fusion protein, 6.6% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition AX comprising: a concentration of 180 mg/mL of a VEGF receptor fusion protein, 7.7% w/v of trehalose, 0.03% w/v of polysorbate 20, and 80 mM of L-arginine hydrochloride, wherein a pH of the aqueous pharmaceutical composition is 5.5-6.4;

an aqueous pharmaceutical composition AY comprising: a concentration of 180 mg/mL of a VEGF receptor fusion protein, 5% w/v of sucrose, 0.01% w/v of polysorbate 20, and 50 mM of L-arginine hydrochloride, wherein a pH of the aqueous pharmaceutical composition is 5.5-6.4;

an aqueous pharmaceutical composition AZ comprising: a concentration of 180 mg/mL of a VEGF receptor fusion protein, 5% w/v of sucrose, 0.05% w/v of polysorbate 20, and 50 mM of L-arginine hydrochloride, wherein a pH of the aqueous pharmaceutical composition is 5.5-6.4; and

an aqueous pharmaceutical composition BA comprising: a concentration of 114.3 mg/mL of a VEGF receptor fusion protein, 8% w/v of trehalose, 0.03% w/v of polysorbate 20, and 20 mM of L-arginine hydrochloride, wherein a pH of the aqueous pharmaceutical composition is 5.5-6.4;

preferably, the VEGF receptor fusion protein is aflibercept.

13. The aqueous pharmaceutical composition according to any one of claims 1-9, wherein the VEGF binding molecule comprises two polypeptides, each polypeptide comprises an immunoglobulin-like domain 2 of VEGFR1 and an immunoglobulin-like domain 3 of VEGFR2.

14. The aqueous pharmaceutical composition according to any one of claims 1-9, wherein the VEGF binding molecule is selected from the group consisting of bevacizumab, ranibizumab, aflibercept, and conbercept.

15. The aqueous pharmaceutical composition according to claim 14, wherein the VEGF binding molecule is aflibercept.

16. The aqueous pharmaceutical composition according to any one of claims 1-15, wherein the aqueous pharmaceutical composition is stable at 2-8°C for at least 6 months, 12 months, or 24 months; or the aqueous pharmaceutical composition is stable at 25 ± 2°C for at least 1 month, 3 months, or 6 months.

17. A lyophilized formulation, wherein the lyophilized formulation is obtained by lyophilizing the aqueous pharmaceutical composition according to any one of claims 1-16, or the lyophilized formulation is reconstituted to obtain the aqueous pharmaceutical composition according to any one of claims 1-16.

18. A delivery device comprising the aqueous pharmaceutical composition according to any one of claims 1-16 or the lyophilized formulation according to claim 17 after reconstitution.

19. The delivery device according to claim 18, wherein the delivery device is a pre-filled syringe.

20. A method for treating an ocular disease or disorder mediated by VEGF, comprising administering the aqueous pharmaceutical composition according to any one of claims 1-16 or the delivery device according to claim 18 or 19 to a subject; preferably, a site of administration is vitreous body.

21. Use of the aqueous pharmaceutical composition according to any one of claims 1-16 or the delivery device according to claim 18 or 19 in the manufacture of a pharmaceutical composition for the treatment of an ocular disease or disorder mediated by VEGF.

22. The method according to claim 20 or the use according to claim 21, wherein the ocular disease or disorder comprises an ocular neovascular disease or an ocular vascular disease.

23. The method or use according to claim 22, wherein the ocular disease or disorder comprises neovascular senile macular degeneration, age-related macular degeneration, macular edema, macular edema after retinal vein occlusion, retinal vein occlusion, central retinal vein occlusion, branch retinal vein occlusion, diabetic macular edema, choroidal neovascularization, iris neovascularization, neovascular glaucoma, postoperative fibrosis of glaucoma, proliferative vitreoretinopathy, optic disc neovascularization, corneal neovascularization, retinal neovascularization, vitreous neovascularization, corneal pannus, pterygium, vascular retinopathy, diabetic retinopathy, non-proliferative diabetic retinopathy, and/or proliferative diabetic retinopathy.

24. A method for delivering a VEGF binding molecule to a subject, comprising a step of administering the aqueous pharmaceutical composition according to any one of claims 1-16 or the delivery device according to claim 18 or 19 to the subject; preferably, a site of administration is vitreous body.

FIG.1

FIG.2

SEC-HPLC（%）

FIG.3

Viscosity（25°C， cP）

FIG.4

FIG.5

FIG.6

## INTERNATIONAL SEARCH REPORT

| International application No. |
|---|
| **PCT/CN2023/119034** |

### A.    CLASSIFICATION OF SUBJECT MATTER

A61K9/00(2006.01)i; A61P27/02(2006.01)i; A61K47/26(2006.01)i; A61K9/08(2006.01)i; A61K38/17(2006.01)i

According to International Patent Classification (IPC) or to both national classification and IPC

### B.    FIELDS SEARCHED

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNTXT, WPABSC, ENTXTC, ENTXT, USTXT, WOTXT, CJFD, VEN, CNKI, DWPI, 万方, WANFANG, 百度, BAIDU, bing, PUBMED, ISI Web of Knowledge, 读秀, DUXIU: 水性, 溶液, 液体, 药物, 组合物, VEGF, 结合分子, VEGF受体, VEGFR, 抗体, 稳定剂, 蔗糖, 海藻糖, 脯氨酸, 赖氨酸, 聚山梨醇酯, 聚山梨酯, 吐温, 泊洛沙姆, 普兰尼克, poloxamer, pluronic, 精氨酸, 表面活性剂, 氯化钠, NaCl, 缓冲液, 无缓冲, buffer, free, free-buffering, 眼药, 眼睛, 玻璃体, aqueous, solution, liquid, medicament, composition, binding molecule, VEGF receptor, antibody, stabilizer, sucrose, trehalose, proline, lysine, polysorbate, arginine, surfactant, sodium chloride, unbuffered, buffer, free, free-buffering, ophthalmic, ocular, vitreous.

### C.    DOCUMENTS CONSIDERED TO BE RELEVANT

| Category* | Citation of document, with indication, where appropriate, of the relevant passages | Relevant to claim No. |
|---|---|---|
| X | CN 112739323 A (REGENERON PHARMACEUTICALS, INC.) 30 April 2021 (2021-04-30) claims 1-38 | 12, 16, 18-24 |
| Y | CN 112739323 A (REGENERON PHARMACEUTICALS, INC.) 30 April 2021 (2021-04-30) claims 1-38 | 1-11, 13-24 |
| Y | CN 114129723 A (QILU PHARMACEUTICAL CO., LTD.) 04 March 2022 (2022-03-04) claims 1-21, description, paragraph 13 | 1-11, 13-24 |
| Y | KAROW, A.R. et al. "Buffer Capacity of Biologics-From Buffer Salts to Buffering by Antibodies" *American Institute of Chemical Engineers*, 01 March 2013 (2013-03-01), abstract | 1-11, 12-24 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **06 December 2023** | **11 December 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/119034** |

**C.    DOCUMENTS CONSIDERED TO BE RELEVANT**

| Category* | Citation of document, with indication, where appropriate, of the relevant passages | Relevant to claim No. |
| --- | --- | --- |
| Y | GARIDEL, P. et al. "Stability of Buffer-Free Freeze-Dried Formulations: A Feasibility Study of a Monoclonal Antibody at High Protein Concentrations" *European Journal of Pharmaceutics and Biopharmaceutics,* Vol. vol. 97, 09 October 2015 (2015-10-09), abstract | 1-11, 13-24 |
| A | CN 103816115 A (CHENGDU KANGHONG BIOTECHNOLOGY CO., LTD.) 28 May 2014 (2014-05-28) entire document | 1-24 |
| A | CN 106999581 A (NOVARTIS AG) 01 August 2017 (2017-08-01) entire document | 1-24 |
| A | WO 2018094316 A1 (JUST BIOTHERAPEUTICS, INC.) 24 May 2018 (2018-05-24) entire document | 1-24 |

Form PCT/ISA/210 (second sheet) (July 2022)

## INTERNATIONAL SEARCH REPORT

| International application No. |
| --- |
| **PCT/CN2023/119034** |

**Box No. II        Observations where certain claims were found unsearchable (Continuation of item 2 of first sheet)**

This international search report has not been established in respect of certain claims under Article 17(2)(a) for the following reasons:

1. ☑  Claims Nos.: **20, 22-23 (in part), 24**
   because they relate to subject matter not required to be searched by this Authority, namely:

   The subject matter of claims 20, 22-23 (in part) and 24 relates to a method for drug treatment of the human or animal body; therefore, no international search is carried out according to PCT Rule 39.1(iv). The search is made on the basis of the use of the aqueous pharmaceutical composition of any one of claims 1-16 or the delivery device of claim 18 or 19 in the preparation of a drug for treating ocular diseases or disorders.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/CN2023/119034** |

| Patent document cited in search report | | | Publication date (day/month/year) | Patent family member(s) | | | Publication date (day/month/year) |
| --- | --- | --- | --- | --- | --- | --- | --- |
| CN | 112739323 | A | 30 April 2021 | CO | 2020014427 | A2 | 21 December 2020 |
| | | | | BR | 112020022610 | A2 | 09 February 2021 |
| | | | | JOP | 20200275 | A1 | 02 November 2020 |
| | | | | CL | 2020002872 | A1 | 26 March 2021 |
| | | | | KR | 20210021299 | A | 25 February 2021 |
| | | | | SG | 11202010684 | YA | 27 November 2020 |
| | | | | JP | 2023071798 | A | 23 May 2023 |
| | | | | MA | 52570 | A | 17 March 2021 |
| | | | | CL | 2021001957 | A1 | 08 April 2022 |
| | | | | JP | 2021523162 | A | 02 September 2021 |
| | | | | JP | 7235770 | B2 | 08 March 2023 |
| | | | | MX | 2020011848 | A | 29 March 2021 |
| | | | | AU | 2019265005 | A1 | 17 December 2020 |
| | | | | WO | 2019217927 | A1 | 14 November 2019 |
| | | | | US | 2021353714 | A1 | 18 November 2021 |
| | | | | PH | 12020551839 | A1 | 28 June 2021 |
| | | | | US | 2019343918 | A1 | 14 November 2019 |
| | | | | US | 11103552 | B2 | 31 August 2021 |
| | | | | EP | 3790532 | A1 | 17 March 2021 |
| | | | | CA | 3099551 | A1 | 14 November 2019 |
| CN | 114129723 | A | 04 March 2022 | None | | | |
| CN | 103816115 | A | 28 May 2014 | None | | | |
| CN | 106999581 | A | 01 August 2017 | SG | 11201702909 | QA | 30 May 2017 |
| | | | | TN | 2017000128 | A1 | 19 October 2018 |
| | | | | JP | 2020193212 | A | 03 December 2020 |
| | | | | KR | 20170076781 | A | 04 July 2017 |
| | | | | KR | 102588846 | B1 | 16 October 2023 |
| | | | | IL | 280087 | A | 01 March 2021 |
| | | | | IL | 280087 | B | 01 February 2022 |
| | | | | TW | 202103735 | A | 01 February 2021 |
| | | | | TWI | 761959 | B | 21 April 2022 |
| | | | | AU | 2020244614 | A1 | 05 November 2020 |
| | | | | AU | 2020244614 | B2 | 01 June 2023 |
| | | | | AU | 2020220210 | A1 | 01 October 2020 |
| | | | | AU | 2020220210 | B2 | 20 January 2022 |
| | | | | RU | 2020114917 | A | 22 May 2020 |
| | | | | RU | 2020114917 | A3 | 17 January 2022 |
| | | | | IL | 251642 | A0 | 29 June 2017 |
| | | | | IL | 251642 | B | 28 February 2021 |
| | | | | BR | 112017008660 | A2 | 26 December 2017 |
| | | | | BR | 112017008093 | A2 | 13 March 2018 |
| | | | | PH | 12017500843 | A1 | 30 October 2017 |
| | | | | MX | 2017005875 | A | 26 June 2017 |
| | | | | KR | 20230066649 | A | 16 May 2023 |
| | | | | MY | 183807 | A | 16 March 2021 |
| | | | | ECSP | 17034829 | A | 28 February 2019 |
| | | | | CA | 2966758 | A1 | 12 May 2016 |
| | | | | JP | 2020079242 | A | 28 May 2020 |
| | | | | JP | 7080263 | B2 | 03 June 2022 |
| | | | | WO | 2016073915 | A1 | 12 May 2016 |

Form PCT/ISA/210 (patent family annex) (July 2022)

## INTERNATIONAL SEARCH REPORT
### Information on patent family members

| International application No. |
| --- |
| **PCT/CN2023/119034** |

| Patent document cited in search report | Publication date (day/month/year) | Patent family member(s) | | | Publication date (day/month/year) |
| --- | --- | --- | --- | --- | --- |
| | | AU | 2015342815 | A1 | 11 May 2017 |
| | | AU | 2015342815 | B2 | 13 December 2018 |
| | | JP | 2017538674 | A | 28 December 2017 |
| | | JP | 6667519 | B2 | 18 March 2020 |
| | | TW | 202146049 | A | 16 December 2021 |
| | | TWI | 806150 | B | 21 June 2023 |
| | | SG | 10201913565 | RA | 27 February 2020 |
| | | IL | 265497 | A | 30 May 2019 |
| | | IL | 265497 | B | 30 June 2021 |
| | | TW | 201625221 | A | 16 July 2016 |
| | | TWI | 738632 | B | 11 September 2021 |
| | | SG | 11201702954 | XA | 30 May 2017 |
| | | EP | 3215122 | A1 | 13 September 2017 |
| | | NZ | 730821 | A | 29 November 2019 |
| | | WO | 2016073918 | A1 | 12 May 2016 |
| | | EP | 3215123 | A1 | 13 September 2017 |
| | | AU | 2018278870 | A1 | 03 January 2019 |
| | | AU | 2018278870 | B2 | 28 May 2020 |
| | | PE | 20170780 | A1 | 04 July 2017 |
| | | US | 2016340420 | A1 | 24 November 2016 |
| | | US | 10689438 | B2 | 23 June 2020 |
| | | AU | 2018274882 | A1 | 20 December 2018 |
| | | AU | 2018274882 | B2 | 09 July 2020 |
| | | US | 2016130337 | A1 | 12 May 2016 |
| | | US | 10035850 | B2 | 31 July 2018 |
| | | JP | 2022141923 | A | 29 September 2022 |
| | | MY | 193913 | A | 01 November 2022 |
| | | EA | 201790989 | A1 | 29 September 2017 |
| | | IL | 283561 | A | 29 July 2021 |
| | | IL | 283561 | B | 01 January 2022 |
| | | CO | 2017004596 | A2 | 31 August 2017 |
| | | PH | 12017500844 | B1 | 30 October 2017 |
| | | CL | 2017001115 | A1 | 26 January 2018 |
| | | IL | 251696 | A0 | 29 June 2017 |
| | | GT | 201700096 | A | 10 October 2019 |
| | | JP | 2017534638 | A | 24 November 2017 |
| | | JP | 6753848 | B2 | 09 September 2020 |
| | | MX | 2017005874 | A | 26 June 2017 |
| | | CL | 2017001117 | A1 | 26 January 2018 |
| | | US | 2020270336 | A1 | 27 August 2020 |
| | | US | 2021340242 | A1 | 04 November 2021 |
| | | AU | 2015342818 | A1 | 11 May 2017 |
| | | AU | 2015342818 | B2 | 03 January 2019 |
| | | RU | 2017119647 | A | 07 December 2018 |
| | | RU | 2017119647 | A3 | 29 May 2019 |
| | | RU | 2722643 | C2 | 02 June 2020 |
| | | KR | 20170082526 | A | 14 July 2017 |
| | | TW | 201625306 | A | 16 July 2016 |
| | | TWI | 705827 | B | 01 October 2020 |
| | | MX | 2021006768 | A | 15 July 2021 |

Form PCT/ISA/210 (patent family annex) (July 2022)

## INTERNATIONAL SEARCH REPORT
### Information on patent family members

International application No.

**PCT/CN2023/119034**

| Patent document cited in search report | | | Publication date (day/month/year) | Patent family member(s) | | | Publication date (day/month/year) |
|---|---|---|---|---|---|---|---|
| | | | | US | 2018298092 | A1 | 18 October 2018 |
| | | | | US | 11098110 | B2 | 24 August 2021 |
| | | | | CA | 2966646 | A1 | 12 May 2016 |
| WO | 2018094316 | A1 | 24 May 2018 | CA | 3043487 | A1 | 24 May 2018 |
| | | | | US | 2022016210 | A1 | 20 January 2022 |
| | | | | EP | 3541365 | A1 | 25 September 2019 |
| | | | | US | 2019298801 | A1 | 03 October 2019 |
| | | | | US | 11135266 | B2 | 05 October 2021 |
| | | | | JP | 2022153566 | A | 12 October 2022 |
| | | | | JP | 2020500195 | A | 09 January 2020 |
| | | | | JP | 7116059 | B2 | 09 August 2022 |

Form PCT/ISA/210 (patent family annex) (July 2022)

**REFERENCES CITED IN THE DESCRIPTION**

**Patent documents cited in the description**

- CN 202211134160
- CN 202211141413
- CN 202311153498
- WO 2005121176 A
- WO 2007112675 A
- WO 2000075319 A1